# EUROPEAN PATENT APPLICATION

(11) **EP 4 790 046 A1**
(43) Date of publication of application: **12.08.2026**
(21) Application number: 24875020.0
(22) Date of filing: 07.10.2024
(51) Int. Cl.: C07C 49/84, C07D 317/50, C07D 307/80, A61K 31/12, A61K 31/36, A61K 31/343, A61K 36/185, A61P 35/00, A23L 33/10

(54) **NOVEL COMPOUND DERIVED FROM MYRISTICA FRAGRANS, AND ANTICANCER COMPOSITION COMPRISING SAME**

(30) Priority: 05.10.2023 KR 20230132691
(71) Applicant: Korea Institute of Science and Technology, Seoul 02792 (KR)
(72) Inventor: JUNG, Sang Hoon, Gangneung-si, Gangwon-do 25451 (KR); KIM, Myungsuk, Gangneung-si, Gangwon-do 25451 (KR); TAM, Thi Le, Gangneung-si, Gangwon-do 25451 (KR); PHUNG, Ngoc Ly, Gangneung-si, Gangwon-do 25451 (KR); LEE, Wookbin, Gangneung-si, Gangwon-do 25451 (KR); KIM, Won Kyu, Gangneung-si, Gangwon-do 25451 (KR)
(74) Representative: Handley, Matthew Edward
(86) International application number: PCT/KR2024/015193
(87) International publication number: WO 2025/075465

(57) **Abstract**

The present invention relates to novel compounds derived from nutmeg and compositions for anticancer use containing the same. In the present invention, various novel compounds derived from nutmeg extract were isolated, and it was confirmed that most of the novel compounds exhibit cytotoxic effects against various cancer cell lines. Five compounds (3, 4, 6, 9, 11) have been found to exhibit high cytotoxic effects against gastric and colorectal cancers. Notably, compound 3 effectively induces apoptosis by regulating the cell cycle of cancer cells and also effectively suppresses tumor growth in xenograft mouse models, making it useful as an anticancer composition.

## Description

### [TECHNICAL FIELD]

The present invention relates to a novel compound derived from *Myristica fragrans* and an anticancer composition containing the same.

### [BACKGROUND ART]

Normal cells can proliferate and inhibit themselves in a regular and elastic manner as needed, whereas cancer cells proliferate uncontrollably. These form masses of undifferentiated cells, also known as tumors. Such cancer cells invade surrounding tissues and metastasize to other organs in the body, causing severe suffering and ultimately leading to death. Despite advances in medicine, the number of cancer patients in Korea has continued to rise, increasing by about 44% over the past decade. Internationally, the anticancer drug market has also grown, reportedly reaching an annual scale of about $100 billion.

However, during or several days after anticancer drug administration, patients may experience loss of appetite, nausea, and vomiting. Immediately after administration or within hours, hypersensitivity reactions (skin rash, angioedema, breathing difficulties) may occur. Further, after chemotherapy administration, side effects such as stomatitis, diarrhea, and neutropenic fever may occur. Therefore, efforts to minimize these side effects are ongoing. As part of these efforts, research utilizing natural products to mitigate the side effects of cancer treatment is actively being conducted.

In addition, nutmeg (*Myristicafragrans*) is an evergreen tree belonging to the Myristicaceae family. Nutmeg refers to what is obtained by harvesting the fruit, removing the seed and drying it, peeling off the hard black seed coat, soaking it overnight in milk of lime, and then drying it at room temperature. Nutmeg has been used to facilitate blood circulation and lung function, and due to its antibacterial effects, it has been employed in the treatment of poor blood flow, respiratory disorders, and skin diseases. Nutmeg also contains aromatic components and is used as a flavoring agent. Research on nutmeg has included reports on its cytotoxic effects on cancer cell lines, antibacterial and antioxidant effects, liver detoxification effects, and inhibition of brain cholinesterase associated with dementia (Korean Patent Publication No. 10-1999-0034285; Korean Patent Publication No. 10-2012-0021283; Korean Patent Publication No. 10-2011-0119483).

### [DISCLOSURE OF THE INVENTION]

### [TECHNICAL PROBLEM]

Accordingly, in the present invention, through extensive research to develop a natural-derived material that reduces anticancer drug side effects and exhibits excellent anticancer effects, a novel compound derived from nutmeg extract was isolated. It has been confirmed that the novel compound exhibits anticancer activity, thereby completing the present invention.

Therefore, the objective of the present invention is to provide a novel compound derived from nutmeg extract.

Another objective of the present invention is to provide a pharmaceutical composition for cancer prevention or treatment, or a health functional food composition for cancer prevention or improvement, containing the compound derived from nutmeg extract as an active ingredient.

### [TECHNICAL SOLUTION]

To achieve the aforementioned objectives, the present invention provides a compound represented by the following Formula A, a pharmaceutically acceptable salt thereof, or an optical isomer thereof: 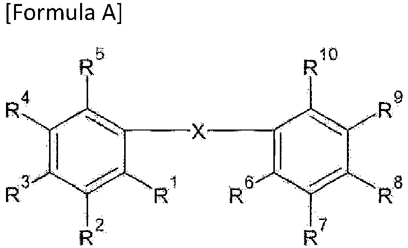
wherein, in Formula A,
X is 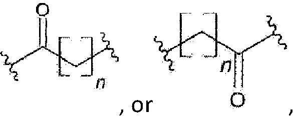 wherein n is an integer of 1 to 20;
at least one of R¹ to R⁵ is 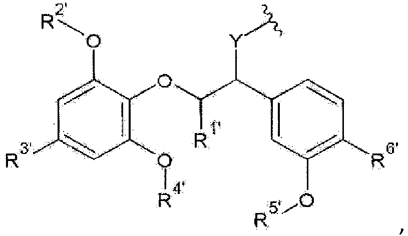 wherein Y is O or - (CH)m- (where m is 0 to 5), and R^{1'} to R^{6'} are each independently hydrogen, hydroxy, optionally substituted C₁₋₆ alkyl, optionally substituted C₁₋₆ alkoxy, optionally substituted C₃₋₈ cycloalkyl, optionally substituted C₂₋₁₀ allyl, or optionally substituted C₃₋₁₀ heterocyclic group;
the remaining R¹ to R⁵ are each independently hydrogen, hydroxy, optionally substituted C₁₋₆ alkyl, optionally substituted C₁₋₆ alkoxy, optionally substituted C₃₋₈ cycloalkyl, optionally substituted C₂₋₁₀ allyl, or optionally substituted C₃₋₁₀ heterocyclic group;
R⁶, R⁸ and R¹⁰ are each independently hydrogen or hydroxy;
R⁷ and R⁹ are each independently hydrogen; and
R^{1'} to R^{6'} are each independently hydrogen, hydroxy, optionally substituted C₁₋₆ alkyl, optionally substituted C1-6 alkoxy, optionally substituted C₃₋₈ cycloalkyl, optionally substituted C₂₋₁₀ allyl, or optionally substituted C₃₋₁₀ heterocyclic group.

In a preferred embodiment of the present invention, the compound represented by the Formula A is a compound represented by the Formula 3; a compound represented by the following Formula 4; a compound represented by the following Formula 6; a compound represented by the following Formula 9; a compound represented by the following Formula 10; a compound represented by the following Formula 11; a compound represented by the following Formula 12; or a compound represented by the following Formula 13:

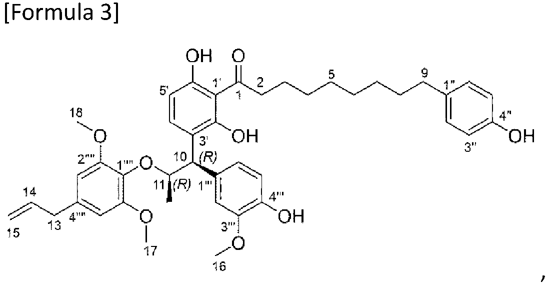

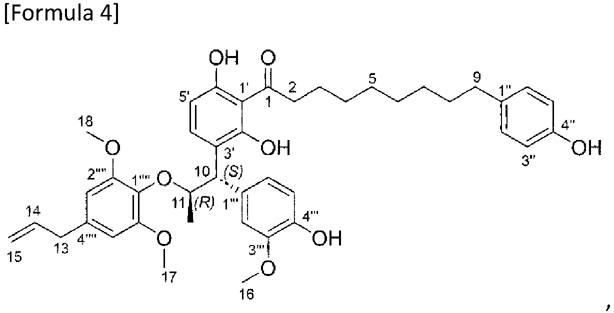

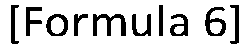

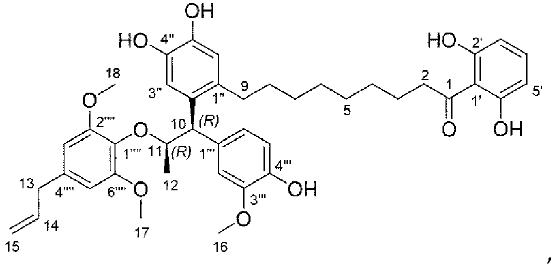

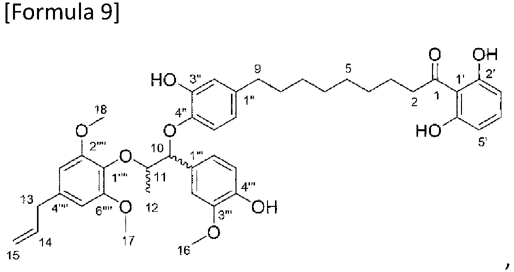

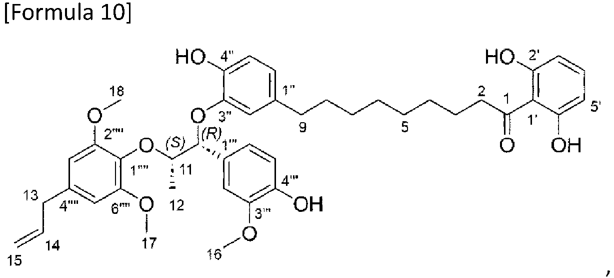

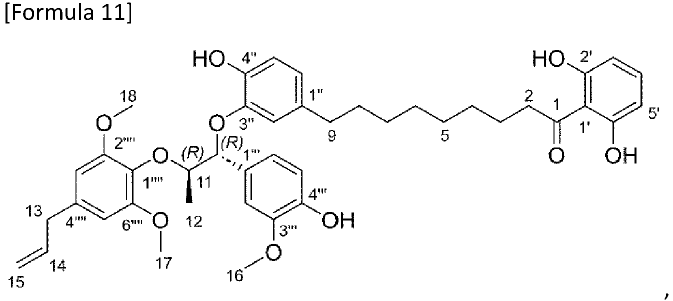

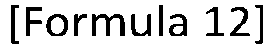

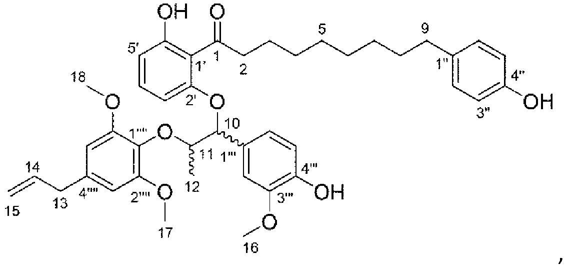

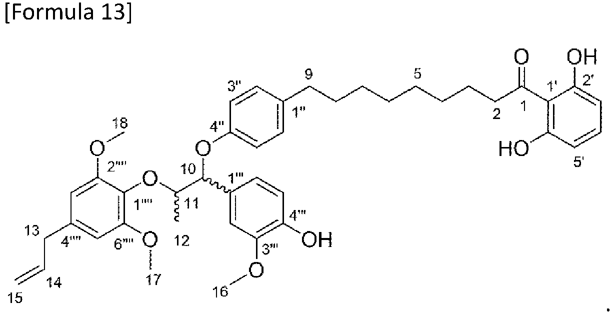

In another preferred embodiment of the present invention, the compound represented by the above Formula A can be a compound represented by the following Formula 3:

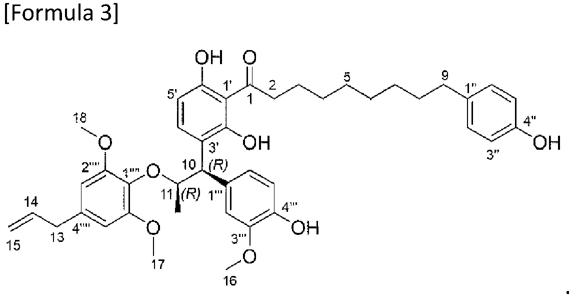

Further, the present invention relates to a compound represented by the following Formula 14, a pharmaceutically acceptable salt thereof, or an optical isomer thereof; a compound represented by the following Formula 15, a pharmaceutically acceptable salt thereof, or an optical isomer thereof; or a compound represented by the following Formula 16, a pharmaceutically acceptable salt thereof, or an optical isomer thereof:

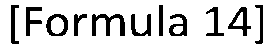

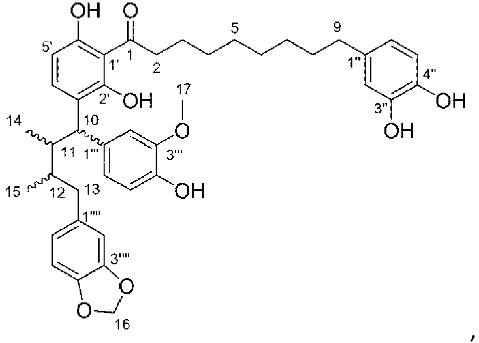

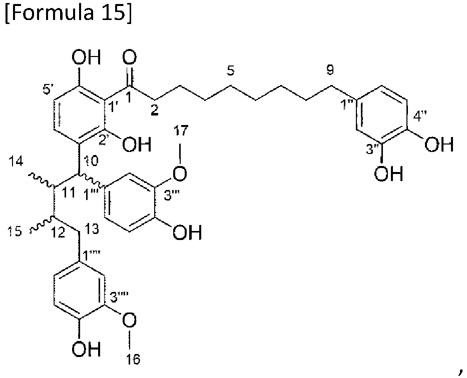

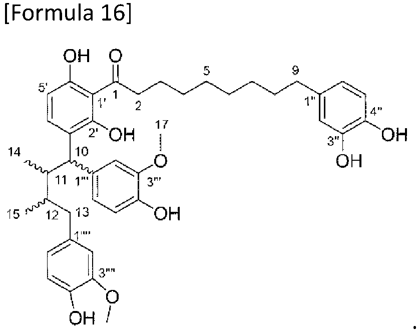

Further, the present invention provides a compound represented by the following Formula 17, a pharmaceutically acceptable salt thereof, or an optical isomer thereof; a compound represented by the following Formula 18, a pharmaceutically acceptable salt thereof, or an optical isomer thereof; a compound represented by the following Formula 19, a pharmaceutically acceptable salt thereof, or an optical isomer thereof; or a compound represented by the following Formula 20, a pharmaceutically acceptable salt thereof, or an optical isomer thereof:

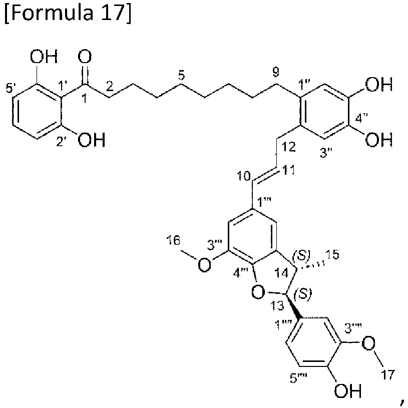

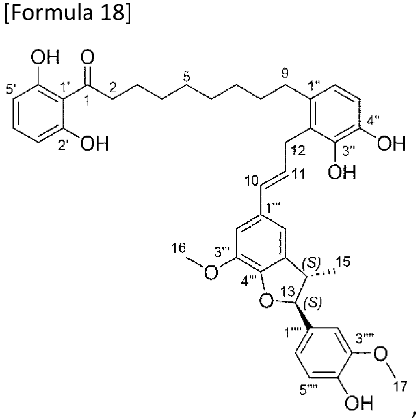

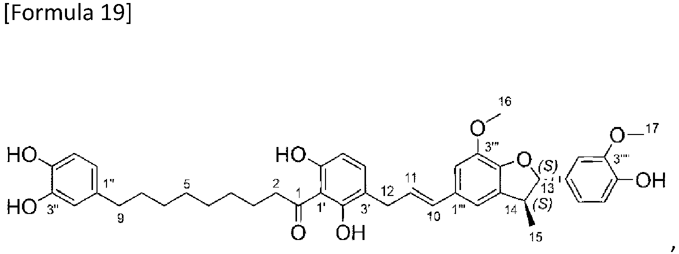

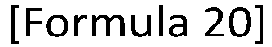

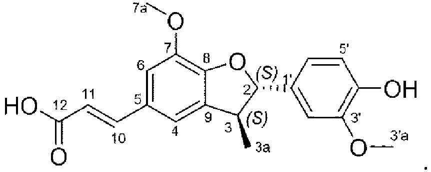

Further, the present invention provides a compound represented by the following Formula 21, a pharmaceutically acceptable salt thereof, or an optical isomer thereof; or a compound represented by the following Formula 22, a pharmaceutically acceptable salt thereof, or an optical isomer thereof:

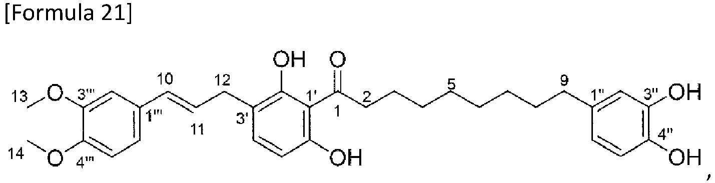

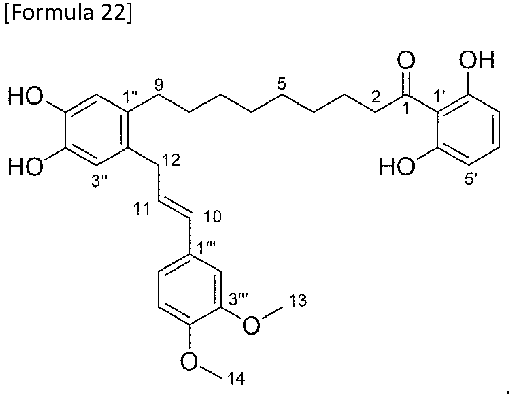

To achieve another objective, the present invention provides a pharmaceutical composition for preventing or treating cancer, or a composition for a health functional food for preventing or improving cancer, containing the above compound as an active ingredient.

In a preferred embodiment of the present invention, the compound can be: a compound represented by the above Formula 3, a pharmaceutically acceptable salt thereof, or an optical isomer thereof; a compound represented by the Formula 4, a pharmaceutically acceptable salt thereof, or an optical isomer thereof; a compound represented by the above Formula 6, a pharmaceutically acceptable salt thereof, or an optical isomer thereof; a compound represented by the above Formula 10, a pharmaceutically acceptable salt thereof, or an optical isomer thereof; a compound represented by the above Formula 11, a pharmaceutically acceptable salt thereof, or an optical isomer thereof; a compound represented by the Formula 12, a pharmaceutically acceptable salt thereof, or an optical isomer thereof; a compound represented by the above Formula 14, a pharmaceutically acceptable salt thereof, or an optical isomer thereof; a compound represented by the above Formula 15, a pharmaceutically acceptable salt thereof, or an optical isomer thereof; a compound represented by the above Formula 17, a pharmaceutically acceptable salt thereof, or an optical isomer thereof; or a compound represented by the Formula 21, a pharmaceutically acceptable salt thereof, or an optical isomer thereof.

### [ADVANTAGEOUS EFFECTS]

In the present invention, various novel compounds derived from nutmeg extract were isolated, and it was confirmed that most of the novel compounds exhibited cytotoxic effects against various cancer cell lines. Five compounds (3, 4, 6, 9, 11) have been found to exhibit high cytotoxic effects against gastric and colorectal cancers. Notably, the compound represented by Formula 3 has been confirmed to effectively induce apoptosis by regulating the cell cycle of cancer cells and to effectively suppress tumor growth in xenograft mouse models, making it useful as an anticancer composition.

### [BRIEF DESCRIPTION OF THE DRAWINGS]

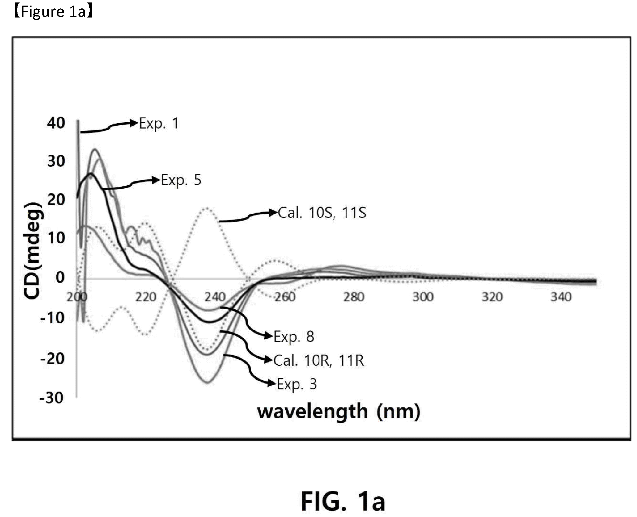

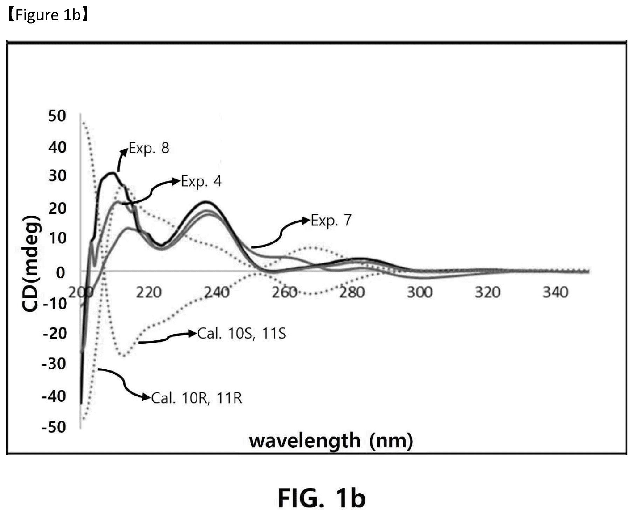

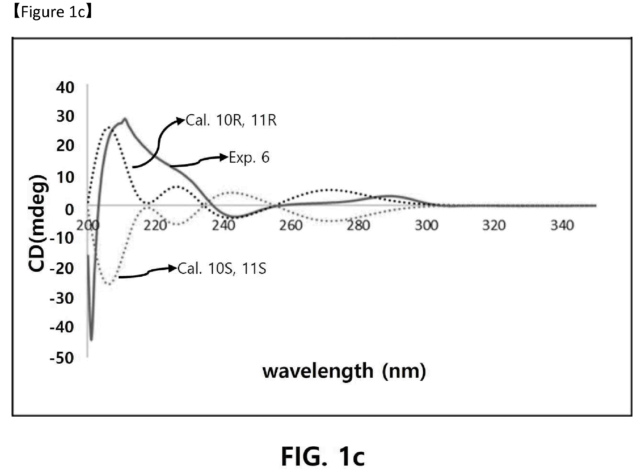

Figures 1a to 1c show data obtained by analyzing the compounds represented by Formulas 1 to 8 using circular dichroism (CD) spectroscopy.

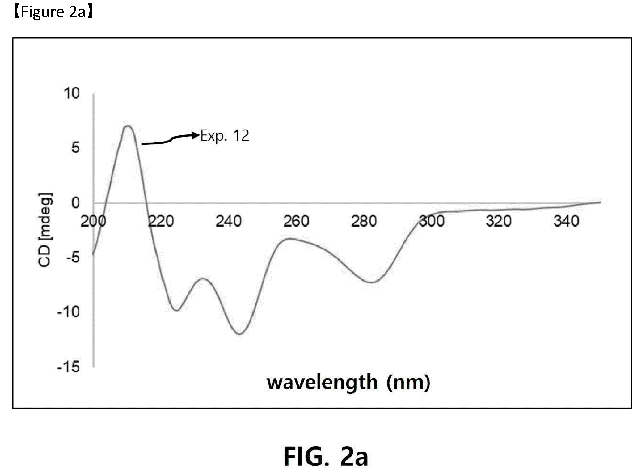

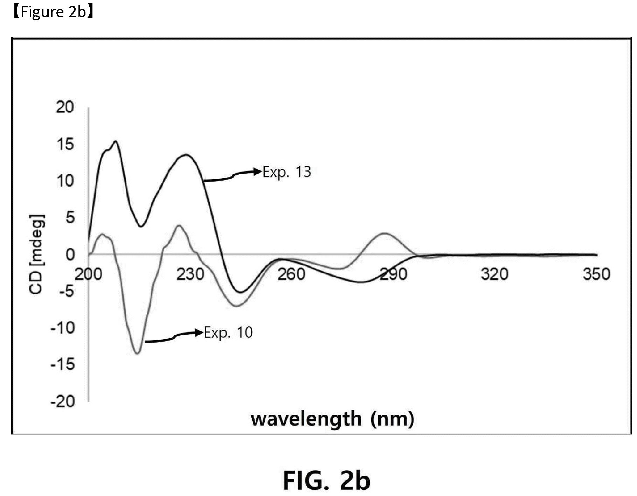

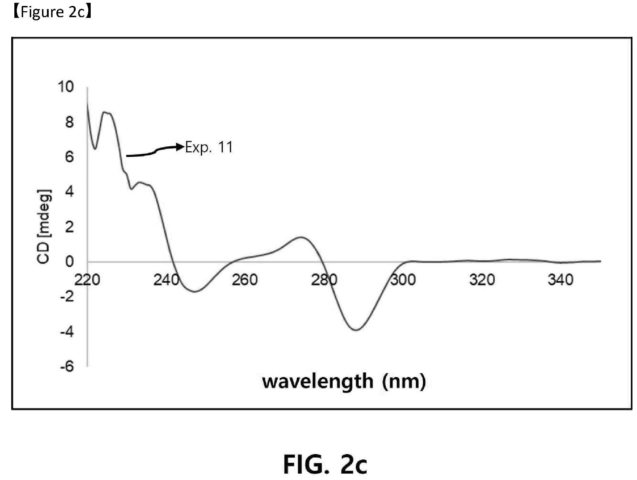

Figures 2a to 2c show data obtained by analyzing the compounds represented by Formulas 10 to 13 using circular dichroism (CD) spectroscopy.

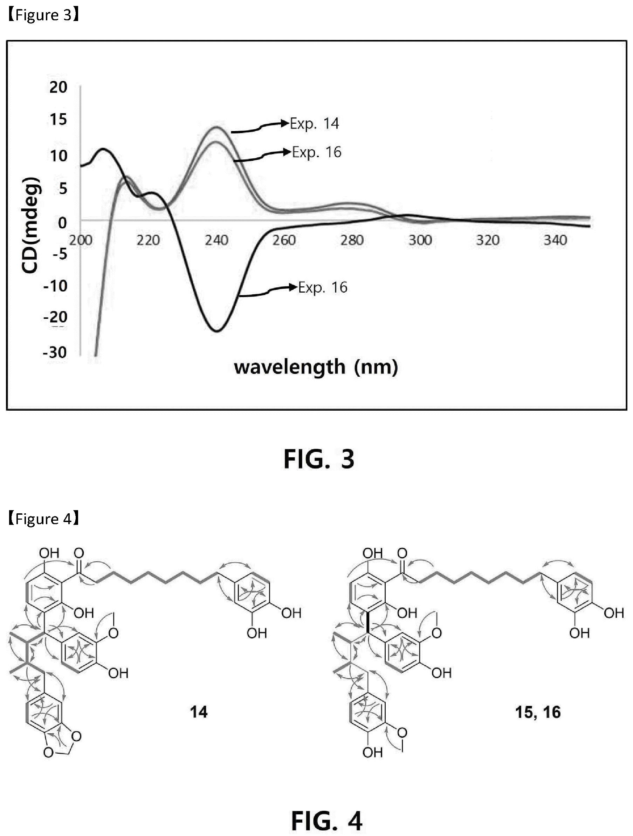

Figure 3 shows data obtained by analyzing the compounds represented by Formulas 14 to 16 using circular dichroism (CD) spectroscopy.
Figure 4 shows data representing the main HMBC (→) and COSY (red, bold) correlations of the compounds represented by Formulas 14 to 16.

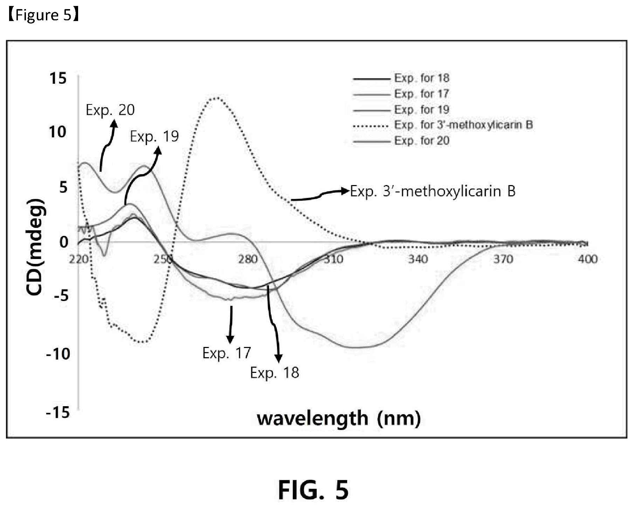

Figure 5 shows data obtained by analyzing the compounds represented by Formulas 17 to 20 using circular dichroism (CD) spectroscopy.

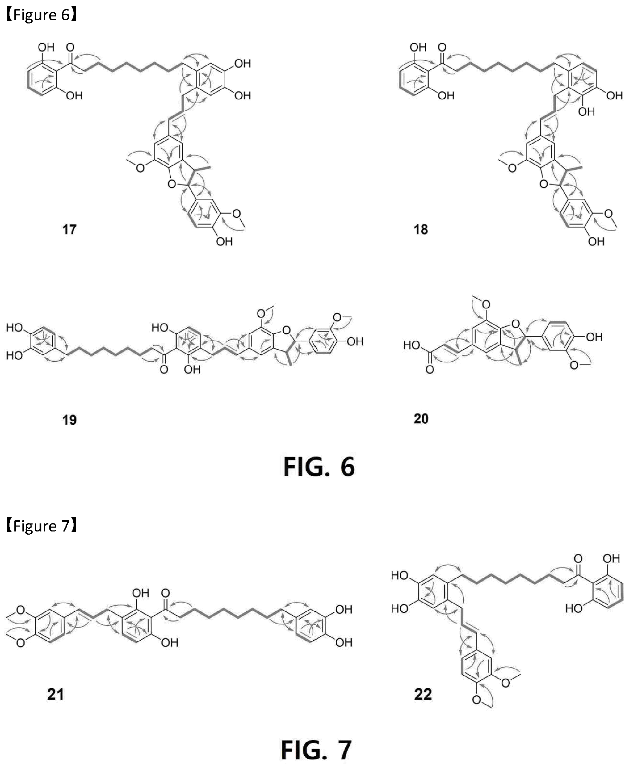

Figure 6 shows data representing the main HMBC (→) and COSY (red, bold) correlations of the compounds represented by Formulas 17 to 20.
Figure 7 shows data representing the main HMBC (→) and COSY (red, bold) correlations of the compounds represented by Formula 21 or 22.
Figure 8 is a heatmap showing the cancer cell line survival rate (%) following treatment with a novel compound (10 µM) derived from nutmeg. The color scale represents the percentage of viable cells relative to the untreated control group, with green indicating higher cell viability and white indicating lower cell viability.
Figure 9 shows data on the cell survival rate (%) when the new compound (10 µM) was applied to gastric cancer cell lines and colorectal cancer cell lines.
Figures 10a and 10b show data on the cell cycle distribution of the 23132/87 and SW48 cell lines when treated with Myricone C and Malabaricone C compounds at 5 µM and 10 µM.
Figures 11a to 11c show data analyzing the cell cycle of 23132/87 and SW48 cell lines when treated with MyrC and MalC compounds at 5 µM and 10 µM.
Figures 12a and 12b show data on the apoptosis patterns in 23132/87 and SW48 cell lines when treated with Myricone C and Malabaricone C compounds at 5 µM and 10 µM.
Figures 13a and 13b show data on the apoptosis distribution (%) in 23132/87 and SW48 cell lines when treated with 5 µM and 10 µM of Myricone C and Malabaricone C compounds.
Figure 14 shows data comparing non-apoptotic cells (Q4: viable cells) with the sum of apoptotic and dead cells (Q2 and Q3) in the 23132/87 and SW48 cell lines.
Figure 15 shows data on caspase-3 and -7 activity in the 23132/87 and SW48 cell lines when treated with Myricone C and Malabaricone C compounds at 5 µM and 10 µM.
Figure 16 shows data on the anticancer effect following administration of the Myricone C compound in a xenograft mouse model. Figure 16a is a schematic diagram illustrating the animal experiment process, Figure 16b shows tumor volume following compound administration, and Figure 16c shows mouse body weight and the weight of major organs (liver, spleen, kidney) following compound administration.

### [MODE FOR THE INVENTION]

The present invention will now be described in detail.

The present invention relates to a compound represented by the following Formula A, a pharmaceutically acceptable salt thereof, or an optical isomer thereof. 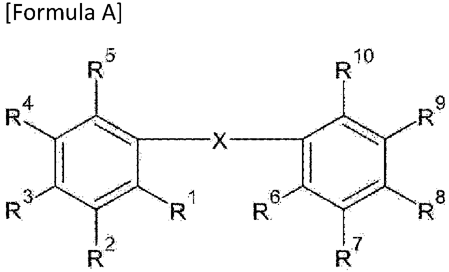
wherein, in Formula A,
X is 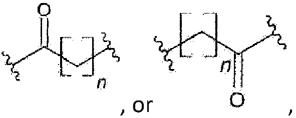 wherein n is an integer of 1 to 20;
at least one of R¹ to R⁵ is 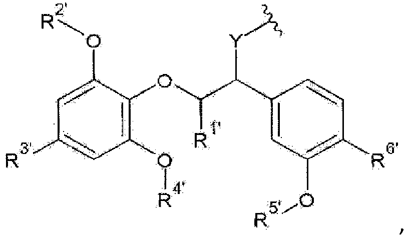 wherein Y is O or - (CH)m- (where m is 0 to 5), and R^{1'} to R^{6'} are each independently hydrogen, hydroxy, optionally substituted C₁₋₆ alkyl, optionally substituted C₁₋₆ alkoxy, optionally substituted C₃₋₈ cycloalkyl, optionally substituted C₂₋₁₀ allyl, or optionally substituted C₃₋₁₀ heterocyclic group;
the remaining R¹ to R⁵ are each independently hydrogen, hydroxy, optionally substituted C₁₋₆ alkyl, optionally substituted C₁₋₆ alkoxy, optionally substituted C₃₋₈ cycloalkyl, optionally substituted C₂₋₁₀ allyl, or optionally substituted C₃₋₁₀ heterocyclic group;
R⁶, R⁸ and R¹⁰ are each independently hydrogen or hydroxy;
R⁷ and R⁹ are each independently hydrogen; and
R^{1'} to R^{6'} are each independently hydrogen, hydroxy, optionally substituted C₁₋₆ alkyl, optionally substituted C1-6 alkoxy, optionally substituted C₃₋₈ cycloalkyl, optionally substituted _{C2-10} allyl, or optionally substituted C₃₋₁₀ heterocyclic group.

In the present invention, the compound represented by the Formula A is a compound represented by the Formula 3; a compound represented by the following Formula 4; a compound represented by the following Formula 6; a compound represented by the following Formula 9; a compound represented by the following Formula 10; a compound represented by the following Formula 11; a compound represented by the following Formula 12; or a compound represented by the following Formula 13:

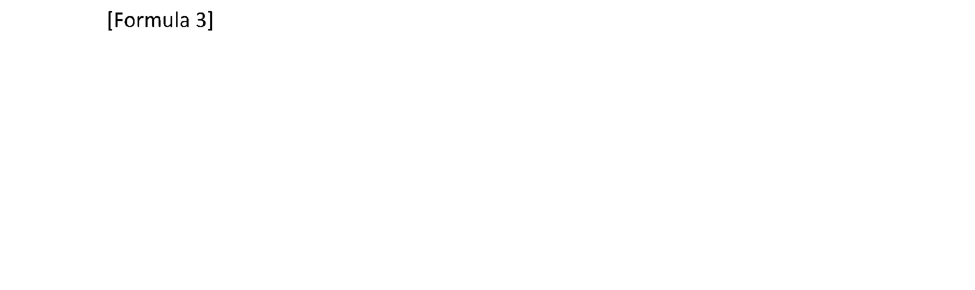

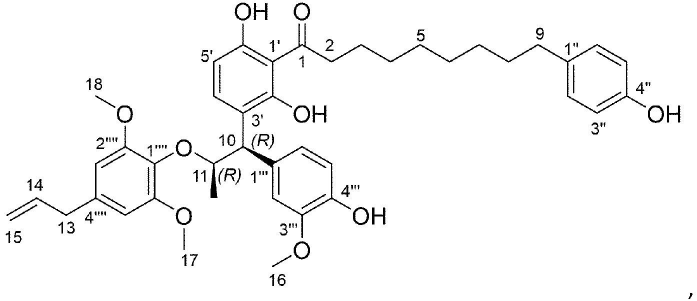

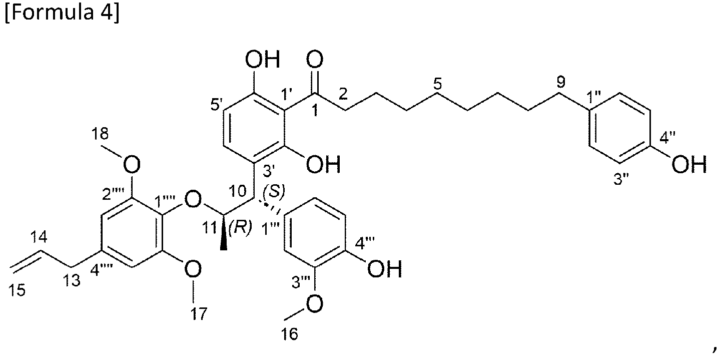

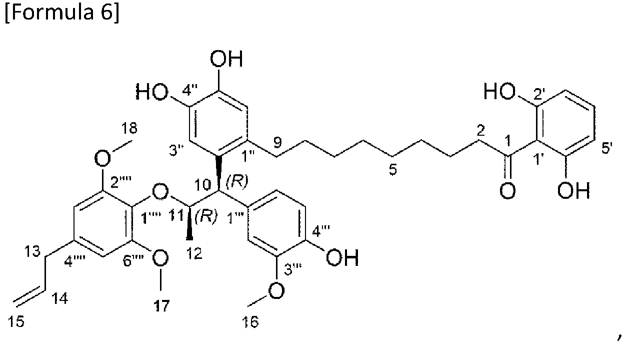

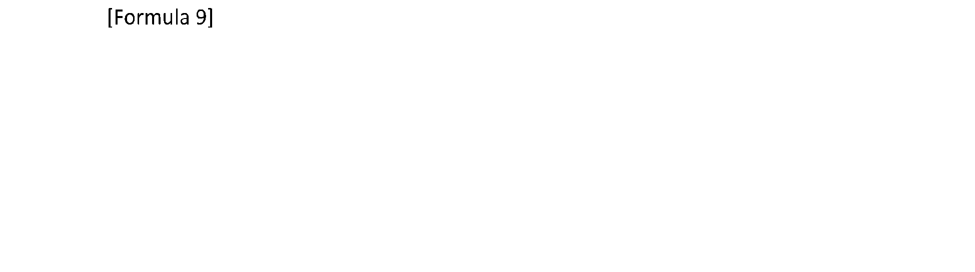

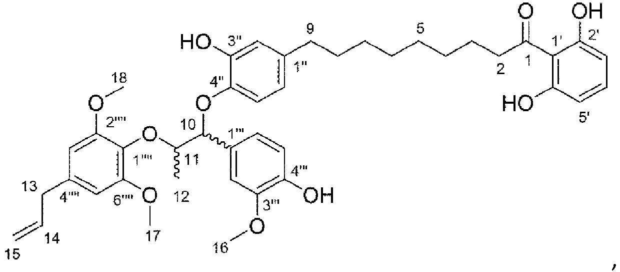

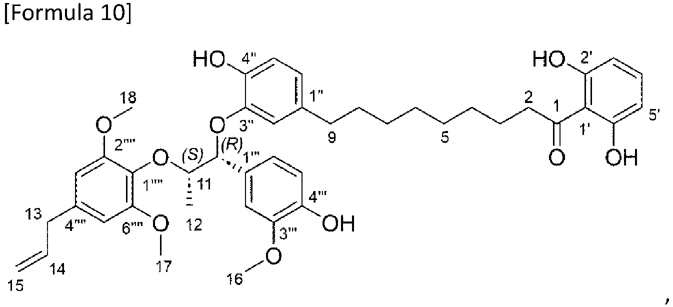

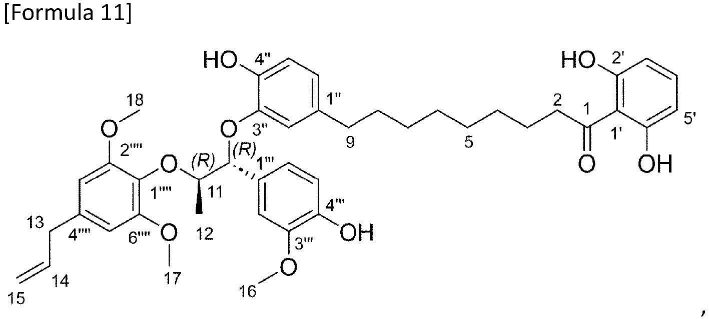

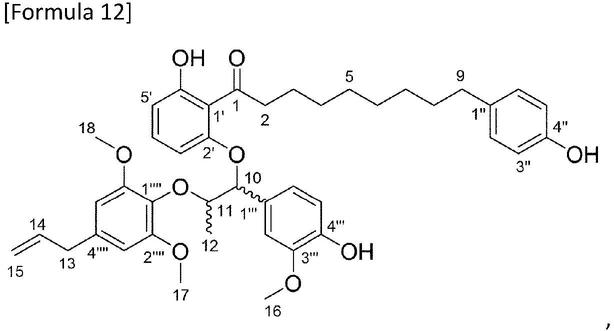

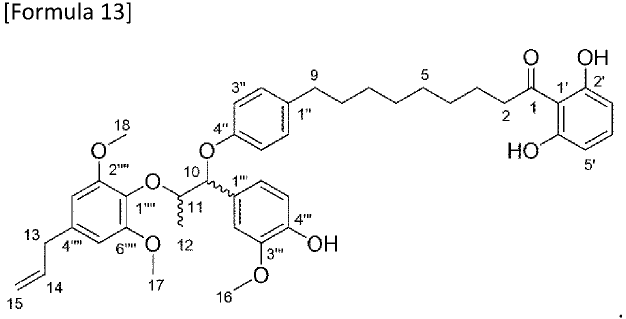

More preferably, the compound represented by the above Formula A can be a compound represented by the following Formula 3:

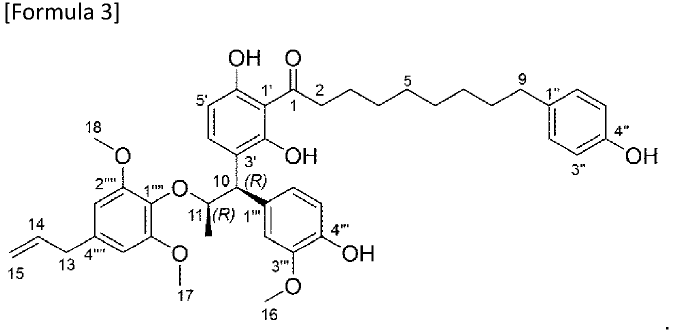

In another aspect, the present invention relates to a compound represented by the following Formula 14, a pharmaceutically acceptable salt thereof, or an optical isomer thereof; a compound represented by the following Formula 15, a pharmaceutically acceptable salt thereof, or an optical isomer thereof; or a compound represented by the following Formula 16, a pharmaceutically acceptable salt thereof, or an optical isomer thereof:

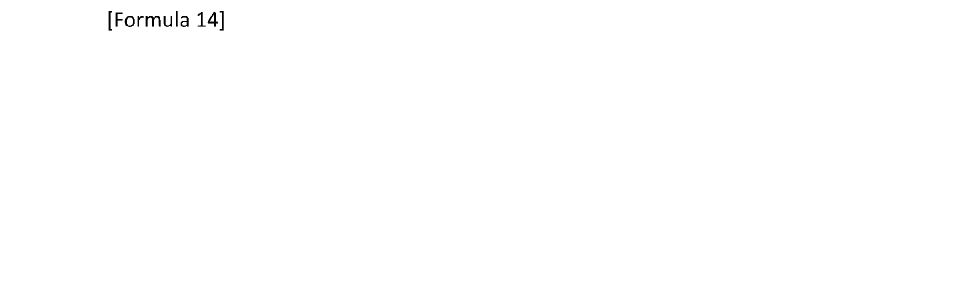

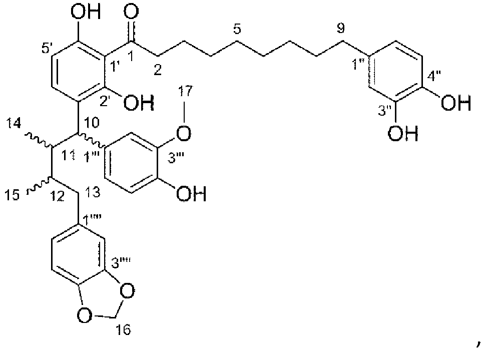

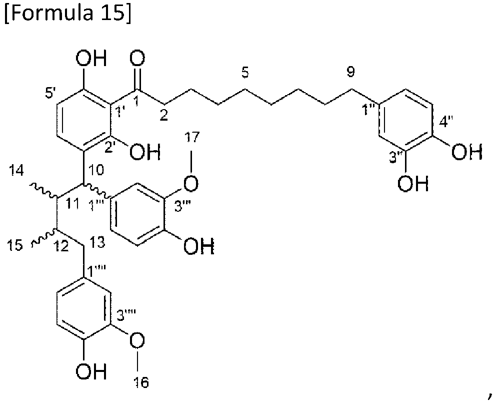

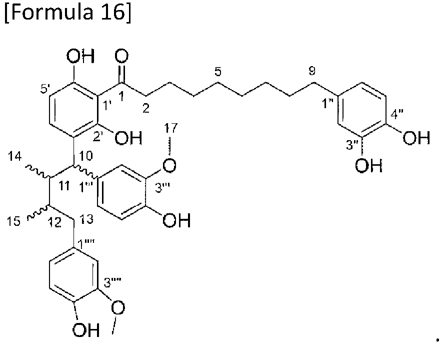

In another aspect, the present invention relates to a compound represented by the following Formula 17, a pharmaceutically acceptable salt thereof, or an optical isomer thereof; a compound represented by the following Formula 18, a pharmaceutically acceptable salt thereof, or an optical isomer thereof; a compound represented by the following Formula 19, a pharmaceutically acceptable salt thereof, or an optical isomer thereof; or a compound represented by the following Formula 20, a pharmaceutically acceptable salt thereof, or an optical isomer thereof:

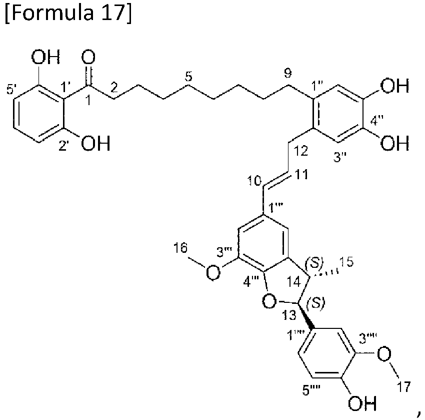

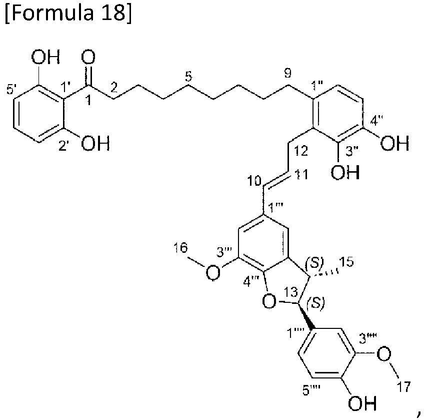

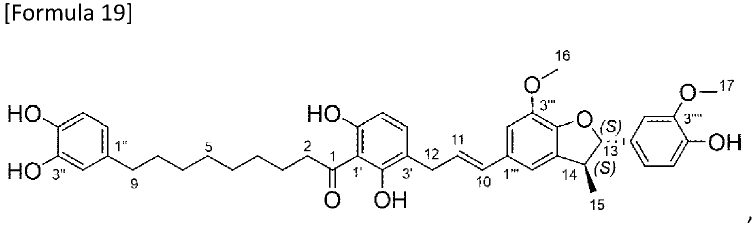

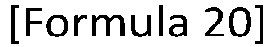

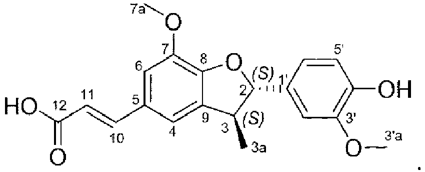

In another aspect, the present invention relates to a compound represented by the following Formula 21, a pharmaceutically acceptable salt thereof, or an optical isomer thereof; or a compound represented by the following Formula 22, a pharmaceutically acceptable salt thereof, or an optical isomer thereof:

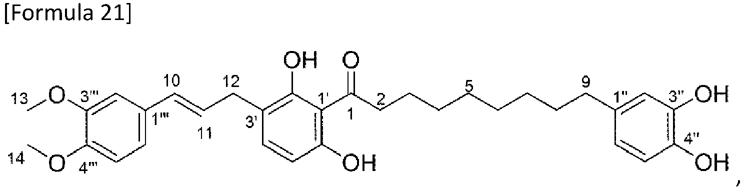

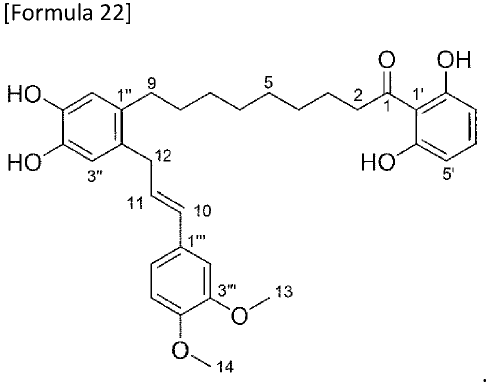

In the present invention, the compound can be isolated from nutmeg (*Myristica fragrans*) extract, and preferably the nutmeg extract can be a 70%(v/v) to 100%(v/v) ethanol extract.

In a specific embodiment of the present invention, 35 compounds were isolated from a 75%(v/v) ethanol extract of nutmeg, among which 17 novel compounds represented by Formula 3, Formula 4, Formula 6, and Formula 9 to Formula 22 were isolated.

In another aspect, the present invention relates to a pharmaceutical composition for preventing or treating cancer, or a composition for a health functional food for preventing or improving cancer, containing the above compounds as active ingredients.

Preferably, the compounds can be a compound represented by the above Formula 3, a pharmaceutically acceptable salt thereof, or an optical isomer thereof; a compound represented by the Formula 4, a pharmaceutically acceptable salt thereof, or an optical isomer thereof; a compound represented by the Formula 6, a pharmaceutically acceptable salt thereof, or an optical isomer thereof; a compound represented by the above Formula 10, a pharmaceutically acceptable salt thereof, or an optical isomer thereof; a compound represented by the above Formula 11, a pharmaceutically acceptable salt thereof, or an optical isomer thereof; a compound represented by the above Formula 12, a pharmaceutically acceptable salt thereof, or an optical isomer thereof; a compound represented by the above Formula 14, a pharmaceutically acceptable salt thereof, or an optical isomer thereof; a compound represented by the above Formula 15, a pharmaceutically acceptable salt thereof, or an optical isomer thereof; a compound represented by the above Formula 17, a pharmaceutically acceptable salt thereof, or an optical isomer thereof; or a compound represented by the Formula 21, a pharmaceutically acceptable salt thereof, or an optical isomer thereof.

In the present invention, the compound can be isolated from nutmeg (*Myristica fragrans*) extract, and preferably the nutmeg extract can be a 70%(v/v) to 100%(v/v) ethanol extract.

In the present invention, the cancer can be pancreatic cancer, liver cancer, lung cancer, stomach cancer, colorectal cancer, prostate cancer, kidney cancer, breast cancer, or ovarian cancer, and preferably can be stomach cancer or colorectal cancer.

In a specific embodiment of the present invention, it was confirmed that most compounds exhibited anticancer activity when novel compounds derived from nutmeg extract was treated with various cancer cell lines. Compounds represented by Formula 3, Formula 4, Formula 6, Formula 10, Formula 11, Formula 12, Formula 14, Formula 15, Formula 17, and Formula 21 exhibited high anticancer activity.

Further, in gastric and colorectal cancers, 5 compounds (Formulas 3, 4, 6, 9, 11) have been found to exhibit high cytotoxic effects. Notably, the compound designated as Formula 3 has been confirmed to effectively induce apoptosis by regulating the cell cycle of cancer cells. Anticancer effects following administration of the Myricone C compound were also confirmed in a xenograft mouse model.

The pharmaceutical compositions of the present invention can be Formulated into various forms using conventional methods. For example, they can be Formulated into oral dosage forms such as powders, granules, tablets, capsules, suspensions, emulsions, and syrups, or into topical preparations, suppositories, and sterile injectable solutions. Pharmaceutically acceptable carriers, excipients, and diluents can be further included according to each Formulation. They may also be Formulated and used as external preparations such as powders, granules, tablets, capsules, suspensions, emulsions, syrups, aerosols, etc., and as sterile injectable solutions using conventional methods.

Suitable carriers, excipients, and diluents include lactose, dextrose, sucrose, oligosaccharides, sorbitol, mannitol, xylitol, erythritol, maltitol, starch, acacia gum, alginate, gelatin, calcium phosphate, calcium silicate, cellulose, methylcellulose, microcrystalline cellulose, polyvinylpyrrolidone, water, methylparaben, propylparaben, talc, magnesium stearate, mineral oil, etc. When formulating or preparing the above pharmaceutical composition, it is prepared using diluents or excipients commonly used, such as fillers, bulking agents, binders, humectants, disintegrants, surfactants, etc.

Solid dosage forms for oral administration include tablets, pills, powders, granules, and capsules. These solid dosage forms are prepared by mixing the composition with at least one excipient, such as starch, calcium carbonate, sucrose, lactose, and gelatin. In addition to simple excipients, lubricants such as magnesium stearate and talc are also used. Liquid Formulations for oral administration include suspensions, solutions, emulsions, and syrups. Besides commonly used simple diluents like water and liquid paraffin, they can contain various excipients such as humectants, sweeteners, flavorings, and preservatives. Formulations for non-oral administration include sterile aqueous solutions, non-aqueous solvents, suspensions, emulsions, freeze-dried preparations, and suppositories. Non-aqueous solvents and suspensions may use propylene glycol, polyethylene glycol, vegetable oils like olive oil, or injectable esters such as ethyl oleate. Excipients for suppositories can include witepsol, macrogol, tween 61, cocoa butter, lauric acid, glycerol gelatin, etc.

The term "administration" as used herein means providing the pharmaceutical composition of the invention to an individual by any suitable method. The pharmaceutical composition of the present invention can be administered at a therapeutically effective dose, which is the amount of the active ingredient or pharmaceutical composition that induces a biological or medical response in tissues, animals, or humans as conceived by researchers, veterinarians, physicians, or other clinicians, namely, the amount that induces alleviation of the symptoms of the disease or disorder being treated. It will be apparent to those skilled in the art that the therapeutically effective dosage and frequency of administration of the pharmaceutical composition of the present invention will vary depending on the desired effect. Therefore, the optimal dosage to be administered can be readily determined by those skilled in the art and can be adjusted based on various factors, including the type of disease, the severity of the disease, the content of the active ingredient and other components contained in the composition, the type of Formulation, the patient's age, weight, general health condition, gender, and diet, the time of administration, the route of administration, the rate of secretion of the composition, the duration of treatment, and concomitant medications. The pharmaceutical composition of the present invention can be administered to an individual via various routes. For example, it can be administered intravenously, intraperitoneally, intramuscularly, intra-arterially, orally, intracardially, intramedullary, intrathecally, transdermally, enterally, subcutaneously, sublingually, or topically, but is not limited thereto. The pharmaceutical composition of the present invention can be administered at an amount of 1 to 10,000 mg/kg/day, and can be administered once daily or divided into multiple doses.

The health functional food composition of the present invention can be used as a health functional food, food additive, or dietary supplement. When using the composition of the present invention as a food additive, it can be used appropriately according to conventional methods, such as adding it as is or mixing it with other foods or food ingredients.

Further, the mixing amount of the health functional food composition can be appropriately adjusted according to the intended use (prevention, health maintenance, or therapeutic treatment). As a specific example, when manufacturing food or beverages, the composition of the present invention is added at an amount of 15% by weight or less, preferably 10% by weight or less, relative to the raw materials. However, when intended for long-term consumption for health and hygiene purposes or for health regulation, it can be added at an amount below the above range. Further, since there are no safety concerns, the active ingredient can be used at amounts exceeding the above range.

There are no particular restrictions on the types of food products, but examples of foods to which the composition of the present invention can be added include meat, sausage, bread, chocolate, candies, snacks, cookies, pizza, instant noodles, other noodle products, chewing gum, dairy products including ice cream, various soups, beverages, tea, drink mixes, alcoholic beverages, vitamin complexes, and all health foods in the conventional sense.

When the health functional food composition of the present invention is manufactured as a beverage, it can contain additional ingredients such as various flavorings or natural carbohydrates, similar to conventional beverages. The natural carbohydrates include monosaccharides such as glucose, and fructose; disaccharides such as maltose and sucrose; natural sweeteners such as dextrin and cyclodextrin; and synthetic sweeteners such as saccharin and aspartame. The natural carbohydrates are included at 0.01 to 10 weight %, preferably 0.01 to 0.1 weight %, relative to the total weight of the food composition of the present invention.

The health functional food composition of the present invention can include various nutritional supplements, vitamins, electrolytes, flavorings, colorants, pectic acids and their salts, alginic acid and its salts, organic acids, protective colloidal thickeners, pH adjusters, stabilizers, preservatives, glycerin, alcohol, carbonating agents used in carbonated beverages, etc. It can include, but is not limited to, pulp for the manufacture of natural fruit juices, fruit juice beverages, and vegetable beverages. These components can be used independently or in combination. The additive ratios are not strictly limited, but it is desirable that they be included within the range of 0.01 to 0.1 weight percent relative to the total weight of the food composition of the present invention.

The present invention will now be described in more detail through the following embodiments.

These examples are provided solely for illustrative purposes and are not intended to limit the scope of the invention. It will be apparent to those skilled in the art that the scope of the invention is not limited by these examples.

### Example 1: Preparation of Nutmeg Extract and Isolation of Compounds

### 1-1: Preparation of Nutmeg Extract

Nutmeg (*Myristica fragrans*) was purchased from Kyung Hee University's herbal medicine department. Five kilograms of nutmeg, chopped into suitable pieces, and 24 liters of 75% ethanol were mixed in an extraction vessel and cold-soaked. The mixture was then filtered using extraction solvent filter paper to obtain the extract. This extraction process was repeated twice. Subsequently, the solvent was concentrated under reduced pressure and dried, yielding 205 g of extract.

### 1-2: Isolation of Compounds Derived from Nutmeg Extract

In the present invention, compounds derived from the nutmeg 75% ethanol extract prepared in <Example 1-1> were isolated.

The extract obtained in <Example 1-1> was sequentially partitioned with methylene chloride (MC) and EtOAc to yield 100 g and 2 g of residue, respectively. The MC-soluble fraction (100 g) was fractionated on a silica gel column (hexane-EtOAc, 20:1 → 1:10) to obtain 35 fractions (1 to 35), and then compounds were isolated from each fraction using HPLC.

**[Table 1]**

| HPLC Analysis Conditions | | |
|---|---|---|
| Time | Acetonitrile (0.1% formic acid) | Water (0.1% formic acid) |
| 0.0 | 5.0 | 95.0 |
| 15.0 | 60.0 | 40.0 |
| 30.0 | 75.0 | 25.0 |
| 42.0 | 90.0 | 10.0 |
| 51.0 | 100.0 | 0.0 |
| 55.0 | 5.0 | 95.0 |
| Column: Triart YMC C18 (Ø4.6x250 mm, 3 µm) | | |
| Flow rate: 0.8 mL/min | | |
| Injection Volume: 10 ul | | |
| Detection wavelength: 280 nm | | |
| Run time: 55 min | | |
| Column Temperature: 30 °C | | |

**[Table 2]**

| Compounds derived from nutmeg extract (1) | | | |
|---|---|---|---|
| No. | Name | Relative configuration | Formula |
| 1 | Myricone A or Myrifragranone B | threo | 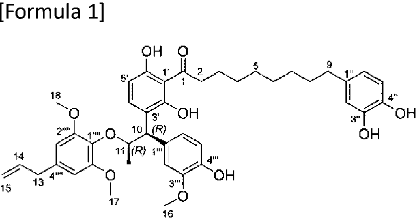 |
| 2 | Myricone B | erythro | 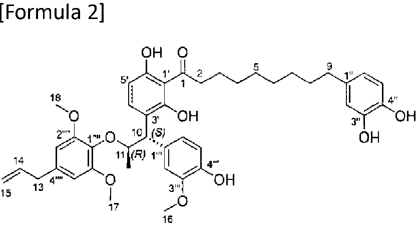 |
| 3 | Myricone C | threo | 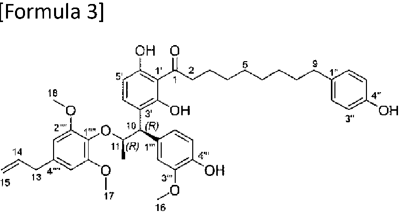 |
| 4 | Myricone D | erythro | 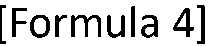 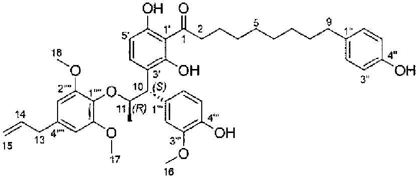 |
| 5 | Myricone E | threo | 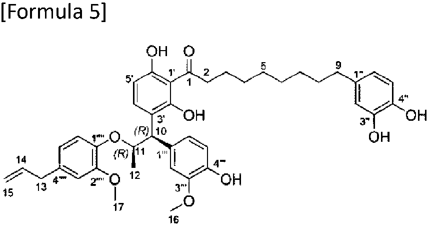 |
| 6 | Myricone F | threo | 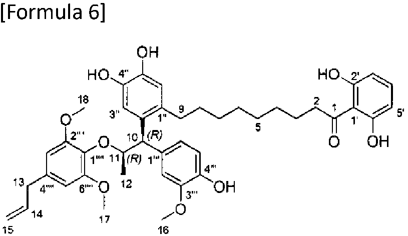 |
| 7 | Myricone G | erythro | 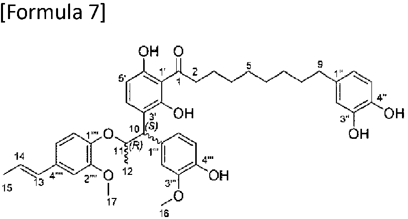 |
| 8 | Myrifragranone A | threo | 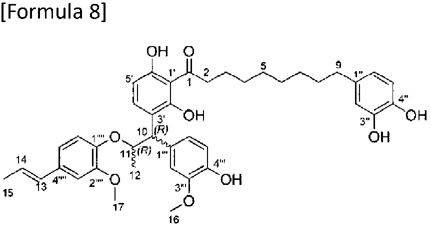 |

**[Table 3]**

| Compounds derived from nutmeg extract (2) | | | |
|---|---|---|---|
| No. | Name | Relative configuration | Formula |
| 9a | Myricone H - erythro form | erythro | 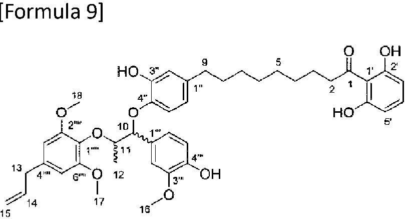 |
| 9b | Myricone I - threo form | threo | |
| 10 | Myricone K | erythro | 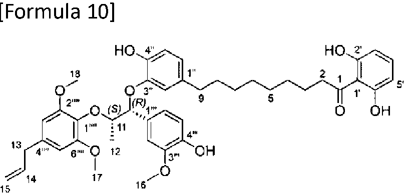 |
| 11 | Myricone L | threo | 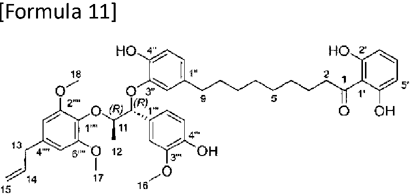 |
| 12 | Myricone M | erythro | 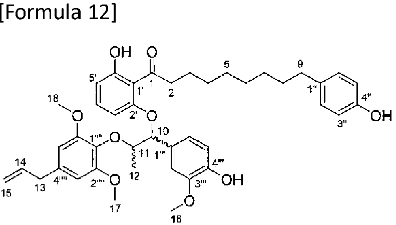 |
| 13 | Myricone N | erythro | 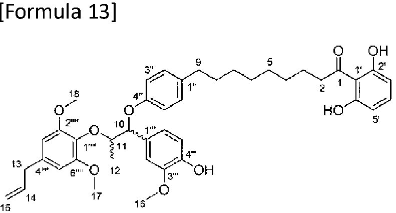 |

**[Table 4]**

| Compounds derived from nutmeg extract (3) | | |
|---|---|---|
| No. | Name | Formula |
| 14 | Myricone O | 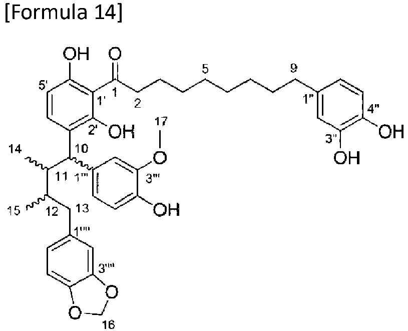 |
| 15 | Myricone P | 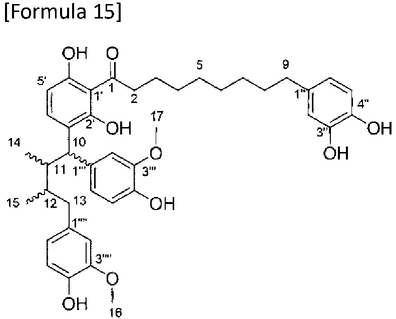 |
| 16 | Myricone R | 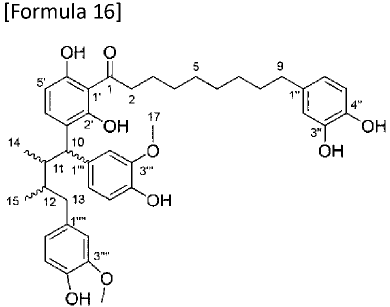 |

**[Table 5]**

| Compounds derived from nutmeg extract (4) | | |
|---|---|---|
| No. | Name | Formula |
| 17 | Myricone Q | 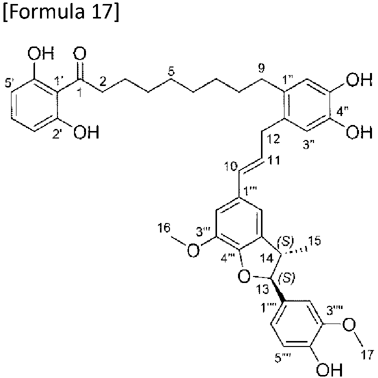 |
| 18 | Myricone T | 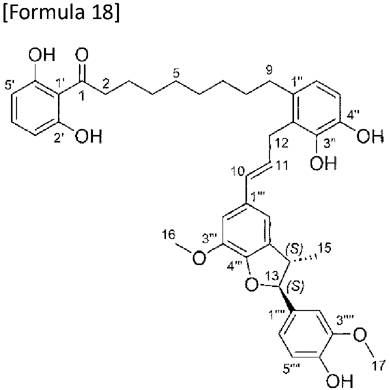 |
| 19 | Myricone X | 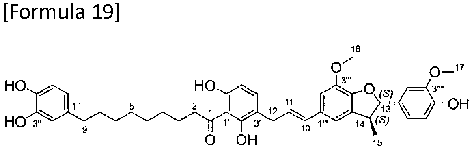 |
| 20 | Myricone Y | 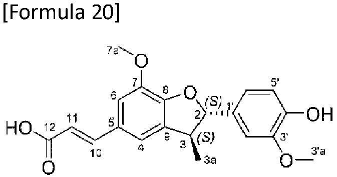 |

**[Table 6]**

| Compounds derived from nutmeg extract (5) | | |
|---|---|---|
| No. | Name | Formula |
| 21 | Myricone Z | 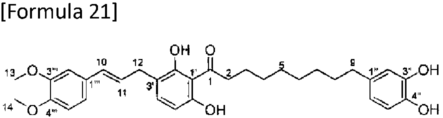 |
| 22 | Myricone W | 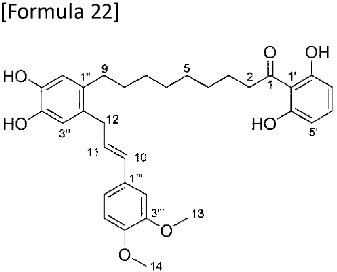 |

**[Table 7]**

| Compounds derived from nutmeg extract (6) | | |
|---|---|---|
| No. | Name | Formula |
| 23 | Malabaricone B | 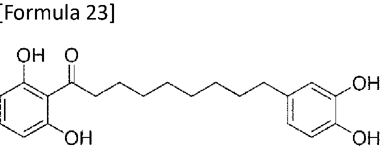 |
| 24 | Malabaricone C | 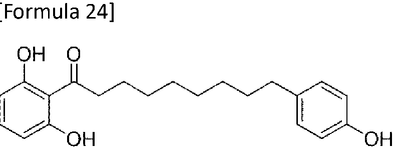 |
| 25 | ProMalabaricone B | 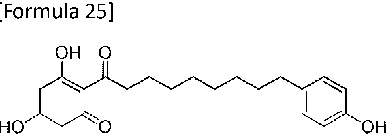 |
| 26 | Giganteones A | 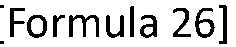 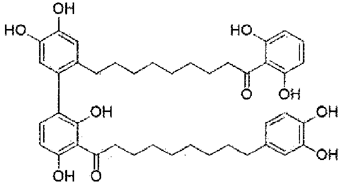 |
| 27 | β-sitosterol | 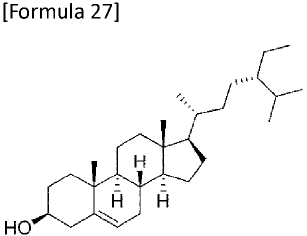 |
| 28 | 3'-Methoxycarin B | 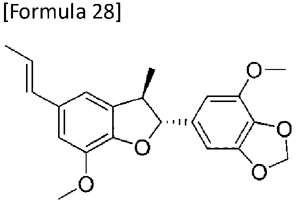 |
| 29 | Myristic acid | 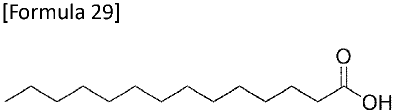 |
| 30 | trans-3,4,5-Trimethoxycinnamic alcohol | 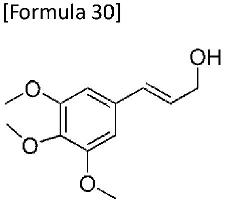 |
| 31 | Myristicin | 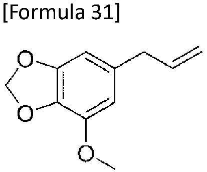 |
| 32 | trans-3,4-dimethoxycinnamyl alcohol | 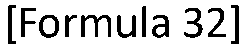 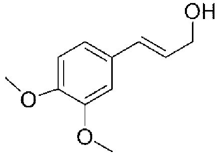 |
| 33 | Anthriscinol | 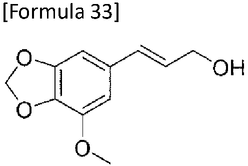 |
| 34 | Methyleugenol | 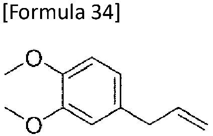 |
| 35 | trans-3,4-Dimethoxycinnamic acid | 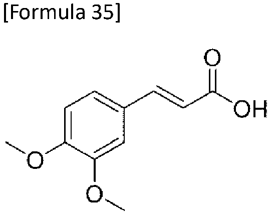 |

In the present invention, 35 compounds were isolated as shown in Tables 2 to 7, and among these, 18 compounds represented by Formulas 3 to 6 and Formulas 9 to 22 have been confirmed to be novel compounds.

Further, the HPLC analysis results for the compounds indicated by Formula 2 to Formula 22 are as shown in Tables 8 to 17 below.

**[Table 8] ¹³C NMR spectroscopic data for the compounds represented by Formulas 2 to 7**

| Position | δ_{C} | | | | | |
|---|---|---|---|---|---|---|
| | 2 | 3 | 4 | 5 | 6 | 7 |
| 1 | 209.22, C | 209.17, C | 208.97, C | 209.35, C | 208.43, C | 209.00, C |
| 2 | 45.10, CH₂ | 45.22, CH₂ | 45.15, CH₂ | 45.43, CH₂ | 44.59, CH₂ | 45.31, CH₂ |
| 3 | 24.56, CH₂ | 24.59, CH₂ | 24.53, CH₂ | 24.74, CH₂ | 24.50, CH₂ | 24.67, CH₂ |
| 4* | 29.28, CH₂ | 29.4, CH₂ | 29.45, CH₂ | 29.48, CH₂ | 29.21, CH₂ | 29.47, CH₂ |
| 5* | 29.26, CH₂ | 29.3, CH₂ | 29.43, CH₂ | 29.45, CH₂ | 29.01, CH₂ | 29.46, CH₂ |
| 6* | 29.14, CH₂ | 29.05, CH₂ | 29.34, CH₂ | 29.32, CH₂ | 28.84, CH₂ | 29.33, CH₂ |
| 7* | 28.88, CH₂ | 29.05, CH₂ | 29.10, CH₂ | 29.04, CH₂ | 28.58, CH₂ | 29.06, CH₂ |
| 8 | 31.39, CH₂ | 31.6, CH₂ | 31.65, CH₂ | 31.56, CH₂ | 30.40, CH₂ | 31.58, CH₂ |
| 9 | 35.14, CH₂ | 34.98, CH₂ | 35.00, CH₂ | 35.30, CH₂ | 31.89, CH₂ | 35.31, CH₂ |
| 10 | 49.86, CH | 53.97, CH | 49.83, CH | 53.92, CH | 52.25, CH | 49.95, CH |
| 11 | 83.26, CH | 80.3, CH | 83.47, CH | 78.44, CH | 79.54, CH | 79.17, CH |
| 12 | 17.87, CH₃ | 18.58, CH₃ | 17.85, CH₃ | 18.97, CH₃ | 18.38, CH₃ | 17.96, CH3 |
| 13 | 40.53, CH₂ | 40.51, CH₂ | 40.53, CH₂ | 40.04, CH₂ | 40.45, CH₂ | 130.62, CH |
| 14 | 137.04, CH | 137.07, CH | 137.03, CH | 137.58, CH | 137.28, CH | 124.88, CH |
| 15 | 116.21, CH₂ | 116.16, CH₂ | 116.21, CH₂ | 116.10, CH2 | 115.94, CH₂ | 18.61, CH₃ |
| 1' | 111.16, C | 112.06, C | 111.23, C | 112.44, C | 110.06, C | 111.44, C |
| 2' | 158.68, C | 158.62, C | 158.52, C | 158.66, C | 161.45, C | 159.04, C |
| 3' | 120.05, C | 119.11, C | 119.72, C | 118.72, C | 108.25, CH | 120.60, C |
| 4' | 137.70, CH | 138.34, CH | 137.68, CH | 138.78, CH | 135.74, CH | 137.43, CH |
| 5' | 108.33, CH | 108.69, CH | 108.43, CH | 109.13, CH | 108.25, CH | 108.61, CH |
| 6' | 161.76, C | 162.63, C | 162.09, C | 162.81, C | 161.45, C | 161.19, C |
| 1" | 135.95, C | 135.08, C | 135.12, C | 136.22, C | 134.49, C | 138.30, C |
| 2" | 115.44, CH | 129.44, CH | 129.44, CH | 115.68, CH | 132.54, C | 115.69, CH |
| 3" | 143.43, C | 115.05, CH | 115.04, CH | 143.55, C | 115.82, CH | 143.54, C |
| 4" | 141.40, C | 153.45, C | 153.43, C | 141.57, C | 141.01, C | 141.51, C |
| 5" | 115.13, CH | 115.05, CH | 115.04, CH | 115.38, CH | 141.36, C | 115.41, CH |
| 6" | 120.69, CH | 129.44, CH | 129.44, CH | 120.97, CH | 116.32, CH | 120.99, CH |
| 1‴ | 133.25, C | 132.35, C | 133.29, C | 132.23, C | 134.85, C | 132.81, C |
| 2‴ | 112.23, CH | 111.55, CH | 112.18, CH | 111.26, CH | 111.75, CH | 112.46, CH |
| 3‴ | 146.49, C | 146.30, C | 146.50, C | 146.62, C | 146.13, C | 146.74, C |
| 4‴ | 144.11, C | 144.11, C | 144.16, C | 144.40, C | 143.60, C | 144.52, C |
| 5‴ | 113.92, CH | 114.02, CH | 113.88, CH | 114.43, CH | 113.96, CH | 114.18, CH |
| 6‴ | 120.95, CH | 121.27, CH | 120.89, CH | 121.00, CH | 121.91, CH | 121.27, CH |
| 1ʺʺ | 133.25, C | 131.49, C | 133.21, C | 144.03, C | 133.42, C | 145.38, C |
| 2ʺʺ | 153.13, C | 153.17, C | 153.13, C | 150.07, C | 153.65, C | 150.36, C |
| 3ʺʺ | 105.37, CH | 105.20, CH | 105.32, CH | 112.58, CH | 105.88, CH | 109.43, CH |
| 4ʺʺ | 136.69, C | 136.56, C | 136.68, C | 134.84, C | 136.61, C | 132.91, C |
| 5ʺʺ | 105.37, CH | 105.20, CH | 105.32, CH | 120.71, CH | 105.88, CH | 118.82, CH |
| 6ʺʺ | 153.13, C | 153.17, C | 153.13, C | 115.28, CH | 153.65, C | 115.96, CH |
| 18 | 55.95, CH₃ | 55.85, CH₃ | 55.93, CH₃ | | 56.11, CH₃ | |
| 17 | 55.95, CH₃ | 55.85, CH₃ | 55.93, CH₃ | 55.79, CH₃ | 56.11, CH₃ | 55.93, CH₃ |
| 16 | 55.95, CH₃ | 55.85, CH₃ | 55.95, CH₃ | **55.94,** CH₃ | 55.90, CH₃ | 56.11, CH₃ |

| | | | | | | |
|---|---|---|---|---|---|---|
| *: data interchangeable | | | | | | |

**[Table 9] ¹H NMR spectroscopic data for the compounds represented by Formulas 2 to 7**

| Position | δ_{H} | | | | | |
|---|---|---|---|---|---|---|
| | 2 | 3 | 4 | 5 | 6 | 7 |
| 2 | 3.22, t (7.4) | 3.18, m | 3.22, t (7.4) | 3.15, t (7.4) | 3.11, td (7.5, 4.6) | 3.18, t (7.4) |
| 3 | 1.73, p (7.3) | 1.68, p (7.4) | 1.72, p (7.2) | 1.65, m | 1.71, p (7.3) | 1.69, m |
| 4-7* | 1.24 - 1.39, m | 1.23 - 1.40, m | 1.22 - 1.41, m | 1.20 - 1.28, m | 1.19 - 1.43, m | 1.20-1.37, m |
| 8 | 1.55, p (7.2) | 1.55, p (7.2) | 1.57, p (7.2) | 1.50, m | 1.41, m; 1.24, m | 1.52, m |
| 9 | 2.48, t (7.6) | 2.52, t (7.7) | 2.53, t (7.7) | 2.44, t (7.6) | 2.42, m | 2.45, t (7.6) |
| 10 | 4.81, d (2.7) | 4.20, d (6.4) | 4.82, d (2.3) | 4.17, d (5.6) | 4.32, d (7.8) | 4.62, d (3.4) |
| 11 | 5.00, qd (6.2, 2.5) | 5.30, p (6.2) | 4.98, qd (6.2, 2.2) | 5.14, p (6.0) | 5.04, p (6.8) | 5.09, m |
| 12 | 1.30, d (6.4) | 1.17, d (6.1) | 1.30, d (6.3) | 1.31, d (6.0) | 1.09, d (6.1) | 1.30, d (6.1) |
| 13 | 3.36, d (6.8) | 3.34, d (6.7) | 3.36, d (6.7) | 3.31, d (6.6) | 3.32, d (6.7) | 6.30, m |
| 14 | 5.96, ddt (16.9, 10.1, 6.7) | 5.96, ddt (16.9, 10.2, 6.7) | 5.96, ddt (16.9, 10.1, 6.7) | 5.92, ddt (16.0, 10.7, 6.6) | 5.95, ddt (16.9, 10.3, 6.7) | 6.11, m |
| 15 | 5.12, m | 5.12, m | 5.13, m | 5.06, m | 5.10, m | 1.84, d (6.5) |
| 3' | | | | | 6.37, d (8.3) | |
| 4' | 6.83, d (8.6) | 7.17, d (8.6) | 6.80, d (8.9) | 7.14, d (8.5) | 7.17, t (8.1) | 6.92, d (8.5) |
| 5' | 6.37, d (8.6) | 6.46, d (8.5) | 6.37, d (8.5) | 6.42, d (8.5) | 6.37, d (8.3) | 6.30, m |
| 2" | 6.70, d (2.0) | 7.04, d (8.1) | 7.05, d (8.5) | 6.66, d (2.0) | | 6.67, m |
| 3" | | 6.75, d (8.4) | 6.76, d (8.8) | | 7.43, s | |
| 5" | 6.77, m | 6.75, d (8.4) | 6.76, d (8.8) | 6.73, m | | 6.73, m |
| 6" | 6.60, dd (8.1, 2.0) | 7.04, d (8.1) | 7.05, d (8.5) | 6.56, dd (8.1, 2.0) | 6.54, s | 6.57, m |
| 2‴ | 6.77, m | 6.88, s | 6.74, m | 6.69, m | 6.83, d (1.9) | 6.79, m |
| 5‴ | 6.88, d (8.1) | 6.88, s | 6.88, d (8.1) | 6.80, d (7.8) | 6.78, d (8.2) | 6.84, m |
| 6‴ | 6.80, dd (8.1, 2.0) | 6.88, s | 6.78, m | 6.73, m | 6.72, dd (8.4, 1.9) | 6.82, m |
| 3ʺʺ | 6.43, s | 6.38, s | 6.42, s | 6.68, m | 6.38, s | 6.86, m |
| 5ʺʺ | 6.43, s | 6.38, s | 6.42, s | 6.69, m | 6.38, s | 6.81, m |
| 6ʺʺ | | | | 6.82, d (7.8) | | 6.81, m |
| 18 | 3.80, s | 3.73, s | 3.80, s | | 3.79, s | |
| 17 | 3.80, s | 3.73, s | 3.80, s | 3.72, s | 3.79, s | 3.81, s |
| 16 | 3.82, s | 3.81, s | 3.83, s | 3.68, s | 3.80, s | 3.79, s |

| | | | | | | |
|---|---|---|---|---|---|---|
| *overlapped | | | | | | |

**[Table 10] ¹³C NMR spectroscopic data for the compounds represented by Formulas 9 to 13**

| Position | δ_{C} | | | | | |
|---|---|---|---|---|---|---|
| | 9a | 9b | 10 | 11 | 12 | 13 |
| 1 | 208.14, C | 208.18, C | 208.33, C | 208.27, C | 208.86, C | 208.11, C |
| 2 | 44.59, CH₂ | 44.61, CH₂ | 44.95, CH₂ | 44.90, CH₂ | 45.99, CH₂ | 45.03, CH₂ |
| 3 | 24.24, CH₂ | 24.27, CH₂ | 24.69, CH₂ | 24.68, CH₂ | 24.32, CH₂ | 24.31, CH₂ |
| 4 | 28.62, CH₂ | 28.64, CH₂ | 29.48, CH₂ | 29.37, CH₂ | 29.82, CH₂ | 29.92, CH₂ |
| 5 | 28.19, CH₂ | 28.22, CH₂ | 29.38, CH₂ | 29.20, CH₂ | 29.56, CH₂ | 29.03, CH₂ |
| 6 | 28.16, CH₂ | 28.16, CH₂ | 29.38, CH₂ | 29.15, CH₂ | 29.32, CH₂ | 28.96, CH₂ |
| 7 | 27.72, CH₂ | 27.71, CH₂ | 28.86, CH₂ | 28.65, CH₂ | 29.30, CH₂ | 28.78, CH₂ |
| 8 | 30.49, CH₂ | 30.49, CH₂ | 31.47, CH₂ | 31.34, CH₂ | 31.84, CH₂ | 31.00, CH₂ |
| 9 | 34.77, CH₂ | 34.77, CH₂ | 35.22, CH₂ | 35.19, CH₂ | 34.85, CH₂ | 34.85, CH₂ |
| 10 | 86.78, CH | 89.67, CH | 86.04, CH | 88.88, CH | 82.77, CH | 80.68, CH |
| 11 | 81.86, CH | 82.02, CH | 81.82, CH | 82.03, CH | 82.06, CH | 83.23, CH |
| 12 | 12.04, CH₃ | 16.34, CH₃ | 12.79, CH₃ | 16.82, CH₃ | 14.55, CH₃ | 14.18, CH₃ |
| 13 | 40.58, CH₂ | 40.58, CH₂ | 40.73, CH₂ | 40.77, CH₂ | 40.75, CH₂ | 40.77, CH₂ |
| 14 | 137.10, CH | 137.10, CH | 137.35, CH | 137.40, CH | 137.36, CH | 137.41, CH |
| 15 | 116.14, CH₂ | 116.16, CH₂ | 116.29, CH₂ | 116.28, CH₂ | 116.28, CH₂ | 116.26, CH₂ |
| 1' | 110.06, C | 110.06, C | 110.29, C | 110.30, C | 112.11, C | 110.31, C |
| 2' | 161.49, C | 161.51, C | 161.56, C | 161.58, C | 161.14, C | 161.58, C |
| 3' | 108.13, CH | 108.07, CH | 108.48, CH | 108.48, CH | 104.84, CH | 108.51, CH |
| 4' | 135.55, CH | 135.55, CH | 135.85, CH | 135.83, CH | 135.49, CH | 135.77, CH |
| 5' | 108.13, CH | 108.07, CH | 108.48, CH | 108.48, CH | 110.54, CH | 108.51, CH |
| 6' | 161.49, C | 161.51, C | 161.56, C | 161.58, C | 164.47, C | 161.58, C |
| 1" | 138.78, C | 138.32, C | 134.55, C | 134.29, C | 135.25, C | 134.71, C |
| 2" | 115.72, CH | 115.09, CH | 118.25, CH | 117.50, CH | 129.61, CH | 129.24, CH |
| 3" | 147.63, C | 147.40, C | 146.66, C | 146.29, C | 115.25, CH | 115.82, CH |
| 4" | 144.85, C | 144.64, C | 145.98, C | 145.75, C | 153.66, C | 156.87, C |
| 5" | 118.84, CH | 118.08, CH | 115.53, CH | 114.90, CH | 115.25, CH | 115.82, CH |
| 6" | 120.03, CH | 119.87, CH | 123.25, CH | 122.83, CH | 129.61, CH | 129.24, CH |
| 1‴ | 130.81, C | 131.29, C | 131.07, C | 131.51, C | 130.12, C | 131.39, C |
| 2‴ | 109.15, CH | 109.34, CH | 109.49, CH | 109.46, CH | 108.66, CH | 109.29, CH |
| 3‴ | 146.63, C | 146.79, C | 146.85, C | 147.04, C | 146.98, C | 146.69, C |
| 4‴ | 145.13, C | 145.67, C | 145.14, C | 145.70, C | 145.31, C | 144.93, C |
| 5‴ | 114.23, CH | 114.36, CH | 114.38, CH | 114.53, CH | 114.76, CH | 114.30, CH |
| 6‴ | 119.65, CH | 120.74, CH | 119.97, CH | 120.95, CH | 119.30, CH | 119.87, CH |
| 1ʺʺ | 132.62, C | 132.91, C | 133.24, C | 133.69, C | 133.78, C | 134.19, C |
| 2ʺʺ | 153.46, C | 153.42, C | 153.62, C | 153.43, C | 153.77, C | 153.70, C |
| 3ʺʺ | 105.25, CH | 105.45, CH | 105.56, CH | 105.68, CH | 105.50, CH | 105.66, CH |
| 4ʺʺ | 136.39, C | 136.44, C | 136.34, C | 136.38, C | 136.25, C | 136.16, C |
| 5ʺʺ | 105.25, CH | 105.45, CH | 105.56, CH | 105.68, CH | 105.50, CH | 105.66, CH |
| 6ʺʺ | 153.46, C | 153.42, C | 153.62, C | 153.43, C | 153.77, C | 153.70, C |
| 18 | 55.89, CH₃ | 55.96, CH₃ | 56.12, CH₃ | 56.16, CH₃ | 55.90, CH₃ | 56.03, CH₃ |
| 17 | 55.89, CH₃ | 55.96, CH₃ | 56.12, CH₃ | 56.16, CH₃ | 55.90, CH₃ | 56.03, CH₃ |
| 16 | 56.05, CH₃ | 56.03, CH₃ | 56.24, CH₃ | 56.23, CH₃ | 56.20, CH₃ | 56.17, CH₃ |

**[Table 11] ¹H NMR spectroscopic data for the compounds represented by Formulas 9 to 13**

| Position | δ_{H} | | | | | |
|---|---|---|---|---|---|---|
| | 9a | 9b | 10 | 11 | 12 | 13 |
| 2 | 3.01, td (7.4, 3.5) | 2.97, td (7.2, 2.9) | 3.07, t (7.4) | 3.09, m | 3.36, m/ 3.76, m | 3.06, m |
| 3 | 1.56, m | 1.56, m | 1.64, m | 1.67, m | 1.73, m | 1.63, m |
| 4-7* | 1.12 - 1.25, m | 1.12 - 1.25, m | 1.11 - 1.33, m | 1.11 - 1.34, m | 1.21 - 1.36, m | 1.16 - 1.38, m |
| 8 | 1.42, p (7.3) | 1.42, p (7.3) | 1.38, m | 1.39, m | 1.52, m | 1.52, m |
| 9 | 2.34, t (7.5) | 2.33, t (8.6) | 2.33, t (7.4) | 2.32, td (7.2, 1.9) | 2.47, t (7.7) | 2.48, m |
| 10 | 5.07, d (2.6) | 4.67, d (8.4) | 5.19, d (2.7) | 4.89, d (8.2) | 5.35, d (2.9) | 5.26, d (3.3) |
| 11 | 4.33, qd (6.5, 2.6) | 4.44, dq (8.0, 6.6) | 4.46, qd (6.4, 2.7) | 4.55, m | 4.33, qd (6.5, 2.8) | 4.28, qd (6.4,3.2) |
| 12 | 1.10, d (6.5) | 0.81, d (6.5) | 1.22, d (6.4) | 0.96, d (6.5) | 1.38, d (6.5) | 1.42, d (6.4) |
| 13 | 3.21, d (6.8) | 3.22, d (6.8) | 3.31, d (6.8) | 3.33, d (6.7) | 3.30, d (6.7) | 3.31, d (6.8) |
| 14 | 5.82, ddt (16.9, 10.3, 6.7) | 5.82, ddt (16.9, 10.3, 6.7) | 5.93, ddt (16.9, 10.1, 6.7) | 5.94, ddt (16.8, 10.0, 6.7) | 5.93, ddt (16.9, 10.1, 6.7) | 5.94, ddt (16.9, 10.0, 6.8) |
| 15 | 4.97, m | 4.97, m | 5.08, m | 5.10, m | 5.07, m | 5.08, m |
| 3' | 6.18, d (8.2) | 6.15, d (8.2) | 6.35, d (8.2) | 6.34, d (8.2) | 6.06, d (8.2) | 6.37, d (8.2) |
| 4' | 6.89, m | 6.89, m | 7.14, d (8.2) | 7.15, d (8.2) | 7.06, d (8.2) | 7.16, d (8.2) |
| 5' | 6.18, d (8.2) | 6.15, d (8.2) | 6.35, d (8.2) | 6.34, d (8.2) | 6.48, d (8.2) | 6.37, d (8.2) |
| 2" | 6.70, d (2.1) | 6.63, d (2.1) | 6.49, d (2.1) | 6.40, d (2.0) | 6.99, d (8.4) | 6.95, d (8.6) |
| 3" | | | | | 6.70, d (8.4) | 6.76, d (8.6) |
| 5" | 6.51, d (8.2) | 6.37, d (8.1) | 6.82, d (8.1) | 6.74, d (8.0) | 6.70, d (8.4) | 6.76, d (8.6) |
| 6" | 6.32, dd (8.3, 2.2) | 6.32, dd (8.3, 2.2) | 6.65, dd (8.2, 2.0) | 6.60, dd (8.1, 1.9) | 6.99, d (8.4) | 6.95, d (8.6) |
| 2‴ | 6.92, d (1.7) | 6.81, m | 7.04, d (1.9) | 6.89, m | 6.72, m | 6.80, d (1.9) |
| 5‴ | 6.78, m | 6.78, m | 6.89, d (8.1) | 6.89, m | 6.85, d (7.9) | 6.80, d (8.1) |
| 6‴ | 6.75, m | 6.79, m | 6.86, dd (8.2, 1.8) | 6.89, m | 6.70, m | 6.72, dd (8.2, 1.8) |
| 3ʺʺ | 6.27, s | 6.30, s | 6.38, s | 6.40, s | 6.33, s | 6.35, s |
| 5ʺʺ | 6.27, s | 6.30, s | 6.38, s | 6.40, s | 6.33, s | 6.35, s |
| 18 | 3.63, s | 3.69, s | 3.73, s | 3.78, s | 3.53, s | 3.58, s |
| 17 | 3.63, s | 3.69, s | 3.73, s | 3.78, s | 3.53, s | 3.58, s |
| 16 | 3.76, s | 3.76, s | 3.86, s | 3.86, s | 3.80, s | 3.77, s |

| | | | | | | |
|---|---|---|---|---|---|---|
| *overlapped | | | | | | |

**[Table 12] ¹³C NMR spectroscopic data for the compounds represented by Formulas 14 to 16**

| Position | *δ_{C}* | | |
|---|---|---|---|
| | 14 | 15 | 16 |
| 1 | 208.70, C | 208.63, C | 208.34, C |
| 2 | 44.93, CH₂ | 44.93, CH₂ | 44.92, CH₂ |
| 3 | 24.69, CH₂ | 24.67, CH₂ | 24.59, CH₂ |
| 4 | 29.36, CH₂ | 29.35, CH₂ | 29.27, CH₂ |
| 5 | 29.32, CH₂ | 29.31, CH₂ | 29.23, CH₂ |
| 6 | 29.24, CH₂ | 29.23, CH₂ | 29.15, CH₂ |
| 7 | 28.98, CH₂ | 28.95, CH₂ | 28.87, CH₂ |
| 8 | 31.52, CH₂ | 31.51, CH₂ | 31.47, CH₂ |
| 9 | 35.26, CH₂ | 35.25, CH₂ | 35.23, CH₂ |
| 10 | 45.87, CH | 45.91, CH | 46.52, CH |
| 11 | 36.94, CH | 36.52, CH | 36.90, CH |
| 12 | 35.03, CH | 35.19, CH | 36.13, CH |
| 13 | 41.99, CH₂ | 41.86, CH₂ | 42.06, CH₂ |
| 14 | 11.11, CH₃ | 11.07, CH₃ | 11.87, CH₃ |
| 15 | 13.33, CH₃ | 13.68, CH₃ | 13.92, CH₃ |
| 1' | 109.98, C | 109.97, C | 110.10, C |
| 2' | 161.51, C | 161.53, C | 161.39, C |
| 3' | 125.47, C | 125.43, C | 124.74, C |
| 4' | 134.08, CH | 134.02, CH | 134.08, CH |
| 5' | 106.61, CH | 106.60, CH | 106.42, CH |
| 6' | 156.82, C | 156.75, C | 156.81, C |
| 1" | 136.33, C | 136.32, C | 136.42, C |
| 2" | 115.66, CH | 115.67, CH | 115.71, CH |
| 3" | 143.53, C | 143.51, C | 143.51, C |
| 4" | 141.50, C | 141.47, C | 141.42, C |
| 5" | 115.38, CH | 115.39, CH | 115.44, CH |
| 6" | 120.97, CH | 121.00, CH | 121.07, CH |
| 1‴ | 136.28, C | 136.49, C | 136.82, C |
| 2‴ | 111.15, CH | 111.22, CH | 111.57, CH |
| 3‴ | 146.53, C | 146.43, C | 146.39, C |
| 4‴ | 143.70, C | 143.65, C | 143.82, C |
| 5‴ | 114.34, CH | 114.15, CH | 114.38, CH |
| 6‴ | 120.66, CH | 120.59, CH | 120.34, CH |
| 1ʺʺ | 135.54, C | 133.77, C | 133.94, C |
| 2ʺʺ | 109.62, CH | 111.57, CH | 111.96, CH |
| 3ʺʺ | 147.55, C | 146.49, C | 146.42, C |
| 4ʺʺ | 145.63, C | 143.61, C | 143.68, C |
| 5ʺʺ | 108.12, CH | 113.98, CH | 114.19, CH |
| 6ʺʺ | 122.08, CH | 121.93, CH | 122.01, CH |
| 16 | 100.90, CH₂ | 55.98, CH₃ | 56.11, CH₃ |
| 17 | 56.00, CH₃ | 55.90, CH₃ | 56.07, CH₃ |

**[Table 13] ¹H NMR spectroscopic data for the compounds represented by Formulas 14 to 16**

| Position | *δ_{H}* | | |
|---|---|---|---|
| | 14 | 15 | 16 |
| 2 | 3.07, t (7.5) | 3.02, t (7.4) | 3.05, t (7.5) |
| 3 | 1.65, m | 1.62, m | 1.66, m |
| 4 - 7 * | 1.24 - 1.38, m | 1.20 - 1.33, m | 1.26 - 1.39, m |
| 8 | 1.54, m | 1.50, m | 1.56, m |
| 9 | 2.47, m | 2.44, m | 2.50, m |
| 10 | 4.18, d (11.9) | 4.11, d (11.9) | 4.05, d (11.8) |
| 11 | 2.26, m | 2.18, m | 2.27, m |
| 12 | 1.84, m | 1.78, m | 1.79, m |
| 13 | 2.47, m | 2.38 - 2.45, m | 2.47, m |
| 14 | 0.74, d (6.7) | 0.70, d (6.8) | 0.74, d (6.7) |
| 15 | 0.85, d (6.8) | 0.84, d (6.6) | 0.84, d (6.8) |
| 4' | 7.14, d (8.5) | 7.06, d (8.4) | 6.81, d (8.4) |
| 5' | 6.21, d (8.4) | 6.16, d (8.4) | 6.11, d (8.4) |
| 2" | 6.70, d (2.0) | 6.66, d (1.9) | 6.73, d (2.0) |
| 5" | 6.77, m | 6.73, d (8.1) | 6.79, d (8.1) |
| 6" | 6.60, dd (8.0, 2.0) | 6.55, m | 6.63, dd (8.1, 1.9) |
| 2‴ | 6.68, d (1.9) | 6.57, m | 6.76, d (1.9) |
| 5‴ | 6.79, d (8.2) | 6.70, d (8.1) | 6.77, d (8.1) |
| 6‴ | 6.65, dd (8.1, 1.9) | 6.55, m | 6.69, dd (8.2, 1.9) |
| 2ʺʺ | 6.55, m | 6.46, d (1.9) | 6.58, d (1.8) |
| 5ʺʺ | 6.75, d (7.7) | 6.81, d (7.9) | 6.87, d (8.0) |
| 6ʺʺ | 6.53, m | 6.55, m | 6.61, dd (8.1, 1.9) |
| 16 | 5.95, s | 3.78, s | 3.86, s |
| 17 | 3.79, s | 3.70, s | 3.84, s |

| | | | |
|---|---|---|---|
| *overlapped | | | |

**[Table 14] ¹³C NMR spectroscopic data for the compounds represented by Formulas 17 to 19**

| Position | δ_{C} | | |
|---|---|---|---|
| | 17 | 18 | 19 |
| 1 | 208.26, C | 208.01, C | 208.42, C |
| 2 | 44.91, CH₂ | 44.98, CH₂ | 45.05, CH₂ |
| 3 | 24.59, CH₂ | 24.62, CH₂ | 24.63, CH₂ |
| 4 | 29.38, CH₂ | 29.65, CH₂ | 29.38, CH₂ |
| 5 | 29.36, CH₂ | 29.61, CH₂ | 29.37, CH₂ |
| 6 | 29.32, CH₂ | 29.62, CH₂ | 29.28, CH₂ |
| 7 | 29.29, CH₂ | 29.58, CH₂ | 29.02, CH₂ |
| 8 | 31.12, CH₂ | 31.73, CH₂ | 31.55, CH₂ |
| 9 | 32.18, CH₂ | 33.10, CH₂ | 35.30, CH₂ |
| 10 | 130.71, CH | 131.04, CH | 131.72, CH |
| 11 | 127.56, CH | 125.76, CH | 125.80, CH |
| 12 | 35.79, CH₂ | 30.04, CH₂ | 33.48, CH₂ |
| 13 | 94.08, CH | 94.10, CH | 94.09, CH |
| 14 | 45.76, CH | 45.75, CH | 45.78, CH |
| 15 | 17.82, CH₃ | 17.83, CH₃ | 17.86, CH₃ |
| 1' | 110.26, C | 110.25, C | 110.31, C |
| 2' | 161.45, C | 161.43, C | 160.40, C |
| 3' | 108.60, CH | 108.59, CH | 119.05, C |
| 4' | 135.90, CH | 135.81, CH | 136.45, CH |
| 5' | 108.60, CH | 108.59, CH | 107.46, CH |
| 6' | 161.45, C | 161.43, C | 158.97, C |
| 1" | 134.09, C | 134.37, C | 136.33, C |
| 2" | 130.77, C | 124.53, C | 115.68, CH |
| 3" | 116.96, CH | 142.53, C | 143.54, C |
| 4" | 141.88, C | 142.04, C | 141.48, C |
| 5" | 141.50, C | 113.27, CH | 115.40, CH |
| 6" | 116.54, CH | 121.61, CH | 121.00, CH |
| 1‴ | 132.04, C | 131.49, C | 131.49, C |
| 2‴ | 109.77, CH | 109.90, CH | 109.81, CH |
| 3‴ | 144.30, C | 144.33, C | 144.41, C |
| 4‴ | 146.89, C | 147.17, C | 147.25, C |
| 5‴ | 133.55, C | 133.58, C | 133.59, C |
| 6‴ | 113.87, CH | 114.04, CH | 114.07, CH |
| 1ʺʺ | 132.18, C | 132.14, C | 132.18, C |
| 2ʺʺ | 109.15, CH | 109.13, CH | 109.12, CH |
| 3ʺʺ | 146.92, C | 146.89, C | 146.89, C |
| 4ʺʺ | 146.00, C | 146.03, C | 146.01, C |
| 5ʺʺ | 114.34, CH | 114.33, CH | 114.33, CH |
| 6ʺʺ | 120.16, CH | 120.17, CH | 120.15, CH |
| 16 | 56.24, CH₃ | 56.26, CH₃ | 56.20, CH₃ |
| 17 | 56.20, CH₃ | 56.20, CH₃ | 56.21, CH₃ |

**[Table 15] ¹H NMR spectroscopic data for the compounds represented by Formulas 17 to 19**

| Position | δ_{H} | | |
|---|---|---|---|
| | 17 | 18 | 19 |
| 2 | 3.07, t (7.5) | 3.05, t (7.4) | 3.09, t (7.5) |
| 3 | 1.65, m | 1.64, m | 1.67, m |
| 4-7* | 1.22 - 1.32, m | 1.22 - 1.34, m | 1.22 - 1.34, m |
| 8 | 1.51, m | 1.52, m | 1.52, m |
| 9 | 2.50, t (7.5) | 2.55, t (7.7) | 2.45, t (7.6) |
| 10 | 6.23 - 6.31, m | 6.36, m | 6.44, d (16.0) |
| 11 | 6.13, dt (15.8, 6.4) | 6.19, dt (15.9, 6.0) | 6.20, dt (15.8, 6.7) |
| 12 | 3.39, d (6.5) | 3.57, dd (6.2, 1.6) | 3.46, dd (6.7, 1.5) |
| 13 | 5.07, d (9.4) | 5.07, d (9.4) | 5.08, d (9.4) |
| 14 | 3.42, m | 3.41, dq (9.5, 6.8) | 3.42, dq (9.4, 6.8) |
| 15 | 1.34, d (6.7) | 1.34, d (6.8) | 1.35, d (6.8) |
| 3' | 6.35, d (8.2) | 6.34, d (8.2) | |
| 4' | 7.17, d (8.2) | 7.17, d (8.2) | 7.15, d (8.3) |
| 5' | 6.35, d (8.2) | 6.34, d (8.2) | 6.31, d (8.2) |
| 2" | | | 6.67, d (2.0) |
| 3" | 6.70, s | | |
| 5" | | 6.72, d (8.2) | 6.73, d (8.0) |
| 6" | 6.69, s | 6.65, d (8.2) | 6.57, dd (8.1, 2.1) |
| 2‴ | 6.76, s | 6.75, s | 6.79, s |
| 6‴ | 6.76, s | 6.75, s | 6.78, s |
| 2ʺʺ | 6.93, s | 6.92, s | 6.93, s |
| 5ʺʺ | 6.87, s | 6.86, s | 6.87, s |
| 6ʺʺ | 6.87, s | 6.86, s | 6.87, s |
| 16 | 3.85, s | 3.84, s | 3.86, s |
| 17 | 3.84, s | 3.84, s | 3.85, s |

| | | | |
|---|---|---|---|
| *overlapped | | | |

**[Table 16] ¹³C NMR & ¹H NMR spectroscopic data for the compounds represented by Formula 20**

| Position | 20 | |
|---|---|---|
| | δ_{C} | δ_{H} |
| 2 | 95.58, CH | 5.18, d (8.9) |
| 3 | 46.70, CH | 3.47, dq (9.6, 7.0) |
| 4 | 118.10, CH | 7.12, s |
| 5 | 130.13, C | |
| 6 | 113.50, CH | 7.14, s |
| 7 | 145.98, C | |
| 8 | 151.25, C | |
| 9 | 135.60, C | |
| 10 | 146.97, CH | 7.66, d (15.8) |
| 11 | 116.70, CH | 6.39, d (15.9) |
| 12 | 171.10, C | |
| 1' | 132.98, C | |
| 2' | 111.04, CH | 7.02, d (1.9) |
| 3' | 149.38, C | |
| 4' | 148.21, C | |
| 5' | 116.31, CH | 6.83, d (8.1) |
| 6' | 120.63, CH | 6.89, dd (8.1, 2.0) |
| 7a | 56.90, CH₃ | 3.91, s |
| 3'a | 56.57, CH₃ | 3.86, s |
| 3a | 18.32, CH₃ | 1.42, d (6.8) |

**[Table 17] ¹³C NMR & ¹H NMR spectroscopic data for the compounds represented by Formulas 21 to 22**

| Position | δ_{C} | | δ_{H} | |
|---|---|---|---|---|
| | 21 | 22 | 21 | 22 |
| 1 | 208.49, C | 208.18, C | | |
| 2 | 45.04, CH₂ | 44.92, CH₂ | 3.09, t (7.4) | 3.07, t (7.4) |
| 3 | 24.65, CH₂ | 24.59, CH₂ | 1.66, m | 1.65, m |
| 4 | 29.38, CH₂ | 29.36, CH₂ | 1.21- 1.36, m* | 1.21 - 1.34, m* |
| 5 | 29.37, CH₂ | 29.34, CH₂ | | |
| 6 | 29.28, CH₂ | 29.30, CH₂ | | |
| 7 | 29.02, CH₂ | 29.26, CH₂ | | |
| 8 | 31.55, CH₂ | 31.11, CH₂ | 1.51, m | 1.51, m |
| 9 | 35.29, CH₂ | 32.19, CH₂ | 2.45, t (7.6) | 2.49, t (7.8) |
| 10 | 131.34, CH | 130.47, CH | 6.38 - 6.45, m | 6.26, d (15.8) |
| 11 | 126.18, CH | 127.84, CH | 6.20, dt (15.8, 6.7) | 6.13, dt (15.8, 6.4) |
| 12 | 33.40, CH₂ | 35.78, CH₂ | 3.46, d (6.7) | 3.39, d (6.4) |
| 13 | 56.16, CH₃ | 56.20, CH₃ | 3.85, s | 3.85, s |
| 14 | 56.04, CH₃ | 56.07, CH₃ | 3.84, s | 3.84, s |
| 1' | 110.29, C | 110.27, C | | |
| 2' | 160.44, C | 161.45, C | | |
| 3' | 119.06, C | 108.62, CH | | 6.36, d (8.2) |
| 4' | 136.48, CH | 135.85, CH | 7.15, d (8.3) | 7.19, d (8.2) |
| 5' | 107.43, CH | 108.62, CH | 6.30, d (8.2) | 6.36, d (8.2) |
| 6' | 158.91, C | 161.45, C | | |
| 1" | 136.32, C | 134.15, C | | |
| 2" | 115.67, CH | 130.67, C | 6.66, d (2.0) | |
| 3" | 143.53, C | 116.95, CH | | 6.70, s |
| 4" | 141.49, C | 141.90, C* | | |
| 5" | 115.39, CH | 141.45, C* | 6.73, d (8.1) | |
| 6" | 121.01, CH | 116.55, CH | 6.57, dd (8.1, 2.0) | 6.69, s |
| 1‴ | 130.50, C | 130.98, C | | |
| 2‴ | 108.87, CH | 108.89, CH | 6.89, d (2.0) | 6.96, d (1.9) |
| 3‴ | 149.19, C | 149.15, C | | |
| 4‴ | 148.78, C | 148.54, C | | |
| 5‴ | 111.38, CH | 111.47, CH | 6.78, d (8.2) | 6.77, d (8.1) |
| 6‴ | 119.52, CH | 119.28, CH | 6.87, dd (8.2, 2.0 ) | 6.84, dd (8.1, 1.9 ) |

| | | | | |
|---|---|---|---|---|
| *overlapped | | | | |

### Example 2: Anticancer Activity of Novel Compounds Derived from Nutmeg Extract

In the present invention, to confirm the anticancer activity of the novel compounds isolated in the above <Examples 1-2>, the novel compounds were tested against gastric cancer cell lines (AGS, 23132/87, SNU-719), colorectal cancer cell lines (SNU-81, SNU-C5 line, SW48), glioblastoma cell lines(SNU201, SNU466), hepatocellular carcinoma cell lines (HepG2), and pancreatic carcinoma cell lines (MiaPaCa-2).

AGS, SW48, HepG2, and MiaPaCa-2 was purchased from ATCC (American Type Culture Collection, USA), SNU cell lines from the Korean Cell Line Bank (KCLB, Korea), and 23132/87 from the Leibniz Institute DSMZ - German Collection of Microorganisms and Cell Cultures GmbH (DSMZ, Germany). All cell lines were cultured in RPMI-1640 medium (HyClone, USA) supplemented with 10% FBS (Gibco, USA) and 1% penicillin-streptomycin (HyClone, USA) under conditions of 5% CO₂, 37°C.

Cells were seeded at a density of 5 × 10³cells/well in a 96-well plate for 24 hours, then treated with the compound at an appropriate concentration (10 µM) for 48 hours. After treatment, alamarBlue reagent was added at a volume ratio of 1/10 of the treatment volume and incubated for 4 hours in a 37°C cell incubator (protected from direct light). Cell viability was then determined by measuring absorbance values at 570 nm and 600 nm using a microplate reader. The anticancer effects of candidate compounds were evaluated by assessing the cell viability (%) of cells treated at specific concentrations and the half-maximal inhibitory concentration (IC₅₀) of each natural product. Doxorubicin and oxaliplatin were used as positive controls for gastric and colorectal cancer, respectively.

All experiments were performed in triplicate, and results are presented as mean ± standard deviation (SD). Statistical analysis was performed using R software (version 4.2.2) (R Core Team). Differences between groups were assessed using student's t-test or 1-way ANOVA followed by post-hoc Tukey HSD test. A p-value < 0.05 was considered statistically significant.

**[Table 18]**

| Cancer cell line survival rate (%) following treatment with a novel compound derived from nutmeg (10 µM) | | | | | | | | | | | |
|---|---|---|---|---|---|---|---|---|---|---|---|
| No. | Comp. | Gastric cancer | | | Colorectal cancer | | | Glioblastoma | | Liver cancer | Pancreat ic cancer |
| | | AGS | 23132/ 87 | SNU-719 | SNU-81 | SNU-C5 | SW48 | SNU20 1 | SNU46 6 | HepG2 | MiaPaCa -2 |
| 1 | Myrifragranone B | 1.13 | 4.71 | 3.95 | 94.79 | 8.73 | 1.75 | 2.07 | 1.7 | 2.07 | 3.15 |
| 2 | Myricone B | 2.45 | 7.44 | 4.55 | 103.32 | 22.12 | 7.98 | 1.43 | 2.29 | 1.47 | 3.67 |
| 3 | Myricone C | 0.16 | 1.77 | 4.30 | 47.08 | 2.11 | 0.01 | 1.17 | 1.91 | 1.60 | 2.53 |
| 4 | Myricone D | 10.15 | 8.41 | 10.19 | 115.60 | 12.41 | 2.61 | 2.44 | 1.74 | 1.84 | 3.54 |
| 5 | Myricone E | 42.09 | 52.10 | 45.03 | 125.80 | 37.58 | 10.17 | 0.61 | 0.58 | 2.34 | 1.64 |
| 6 | Myricone F | 0.51 | 1.46 | 1.36 | 55.77 | 3.53 | 0.82 | 1.4 | 1.96 | 1.32 | 2.16 |
| 8 | Myrifragranone A | 2.19 | 3.92 | 2.27 | 10.02 | 4.41 | 2.04 | 108.12 | 93.39 | 95.41 | 74.57 |
| 9a,9b | Myricone H, I | 102.50 | 95.11 | 102.75 | 105.34 | 73.12 | 86.32 | 70.93 | 36.08 | 31.19 | 45.95 |
| 10 | Myricone K | 4.14 | 2.77 | 1.85 | 16.06 | 4.94 | 2.67 | 3.28 | 0.6 | 8.70 | 4.30 |
| 11 | Myricone L | 20.32 | 19.04 | 12.53 | 92.53 | 13.37 | 5.57 | 32.75 | 0.52 | 25.27 | 22.04 |
| 12 | Myricone M | 7.89 | 0.67 | 0.53 | 2.27 | 1.98 | 2.52 | 0.75 | 0.64 | 1.23 | 3.46 |
| 14 | Myricone O | 81.41 | 78.21 | 71.59 | 103.66 | 18.76 | 31.12 | 100.61 | 56.11 | 67.05 | 87.78 |
| 15 | Myricone P | 8.49 | 1.45 | 2.14 | 6.63 | 2.62 | 3.93 | 31.39 | 0.99 | 3.18 | 4.32 |
| 17 | Myricone Q | 80.52 | 85.02 | 54.85 | 113.08 | 58.45 | 60.21 | 105.07 | 45.59 | 108.13 | 59.90 |
| 18 | Myricone T | 91.90 | 87.08 | 104.28 | 102.20 | 78.03 | 83.45 | 104.25 | 86.19 | 89.04 | 74.78 |
| 19 | Myricone X | 84.50 | 89.28 | 107.97 | 101.62 | 69.64 | 69.66 | 106.52 | 80.93 | 52.38 | 56.93 |
| 21 | Myricone Z | 6.86 | 2.40 | 4.60 | 33.61 | 6.53 | 3.51 | 67.76 | 0.95 | 6.05 | 7.58 |

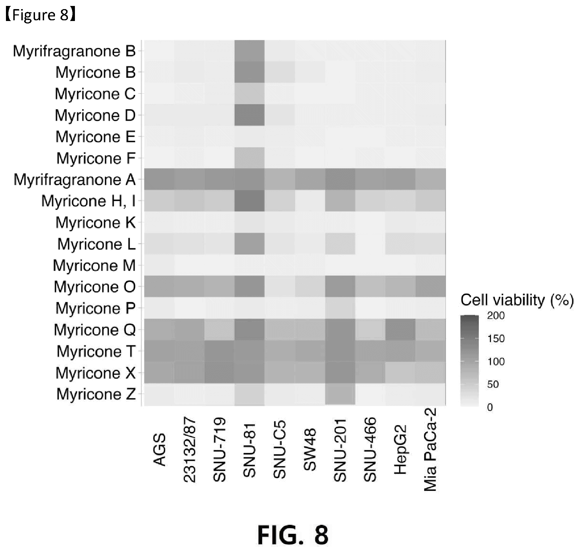

As a result, as shown in Table 18 and Figure 8, it was confirmed that most of the new compounds isolated from nutmeg exhibit anticancer activity. In particular, 5 compounds (3, 4, 6, 9, 11) have been found to show excellent cytotoxic effects in gastric cancer cell lines and colorectal cancer cell lines.

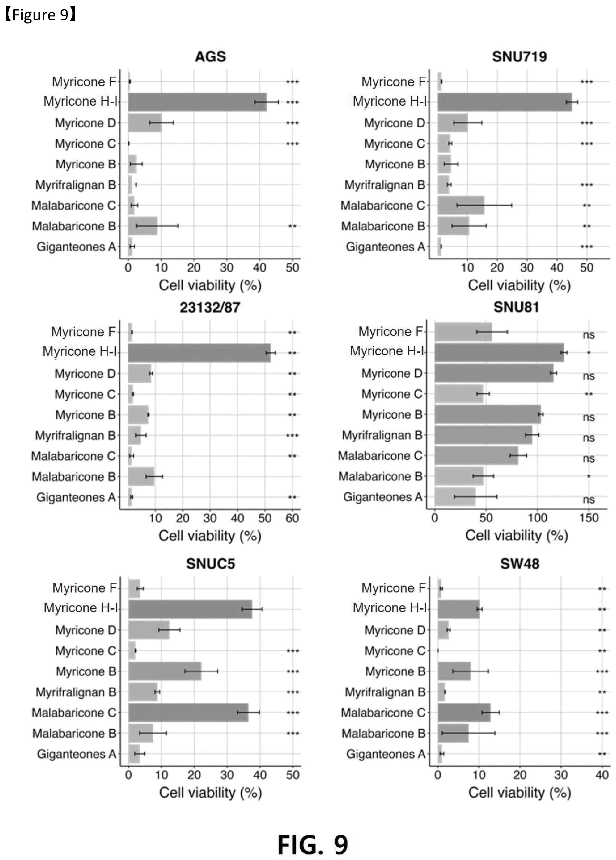

Generally, most compounds showed effects starting at 10 µM treatment, but SNU81 cells exhibited higher resistance to the compounds. However, the response of these compounds to SNU81 cells were superior to that of the positive control, oxaliplatin, and the compound response to SNU-C5 cells were also similar to that of oxaliplatin (Figure 9).

Further, to measure the IC₅₀ values against six cancer cell lines. Each cell line was treated with the new compound at various concentrations (2.5-40 µM), and the IC₅₀ values were measured.

**[Table 19]**

| IC₅₀ Values of Compounds for Six Cancer Cell Lines (µM) | | | | | | |
|---|---|---|---|---|---|---|
| Compound | Gastric cancer | | | Colorectal cancer | | |
| | AGS | SNU719 | 23132/8 7 | SNU81 | SNUC5 | SW48 |
| Myricone F (Formula 6) | 3.35 | 2.58 | 2.39 | 10.30 | 4.04 | 3.09 |
| Myricone H-I Mixture (Formula 9a-9b) | 8.88 | 8.78 | 10.78 | 39.80 | 7.88 | 4.73 |
| Myricone D (Formula 4) | 7.27 | 5.01 | 4.14 | 16.87 | 5.57 | 5.73 |
| Myricone C (Formula 3) | 3.62 | 2.93 | 2.07 | 9.84 | 2.54 | 2.55 |
| Myricone B (Formula 2) | 4.59 | 4.70 | 4.25 | 16.58 | 6.85 | 6.18 |
| Myrifragranone B (Formula 1) | 4.07 | 3.99 | 4.09 | 14.64 | 6.32 | 4.30 |
| Malabaricone C (Formula 24) | 5.79 | 7.30 | 6.37 | 12.20 | 9.76 | 6.79 |
| Malabaricone B (Formula 23) | 6.15 | 5.93 | 4.79 | 9.31 | 4.88 | 4.93 |
| Giganteones A (Formula 26) | 1.71 | 4.60 | 4.08 | 8.94 | 3.72 | 2.77 |
| Positive control | 1.50 | 2.57 | 0.45 | 41.49 | 12.55 | 2.14 |

The IC₅₀ values of the compounds are presented in Table 19. Among the new compounds, Myricone C and Myricone G exhibited the strongest anticancer activity, showing the lowest IC₅₀ values. As mentioned earlier, the IC values of Myricone C and Myricone G against SNU81 and SNUC5 cells were 9.84 µM and 2.54 µM, and 10.30 µM and 4.04 µM, respectively, significantly lower than the IC₅₀ values of oxaliplatin (41.49 µM and 12.55 µM). In contrast, the mixture of Myricone E-F exhibited the weakest effect, with IC₅₀ values at least 1.5-fold to 5-fold higher than the most potent candidate in each cell line. Among the existing compounds, Giganteones A showed overall superior activity to Myrifragranone B and Malabaricone C in most cell lines.

Based on this analysis, the new compound Myricone C and the existing compound Malabaricone C were selected for in-depth testing against representative cell lines of each cancer type: 23132/87 (gastric cancer) and SW48 (colorectal cancer).

### Example 3: Verification of the Effect of the Myricone C Compound on the Cancer Cell Cycle

### 3-1: Verification of Cell Cycle Following Myricone C Treatment

One characteristic of cancer is its ability to evade cell cycle regulation and divide rapidly. Therefore, the present invention analyzed the cell cycles of the 2 cancer cell lines in the presence and absence of Myricone C or Malabaricone C.

Cell cycle distribution was analyzed by DNA staining using FxCycle PI/RNase Staining Solution (Invitrogen, USA). The 23132/87 and SW48 cell lines was seeded at densities of 5 × 10⁵ and 7 × 10⁵cells/well, respectively, in 6-well plates. After 24 hours of culture, cells were starved overnight in serum-free medium. Subsequently, cells were treated with Myricone C or Malabaricone C at 2 concentrations (5 and 10 µM) or DMSO (control) for 48 hours. Cells were then harvested, fixed with 70% ethanol, and stained with PI/RNase solution. Samples were analyzed by flow cytometry (BD FACSVerse Cell Analyzer and FlowJo software, BD Biosciences, Franklin Lakes, NJ, USA) based on 10,000 events.

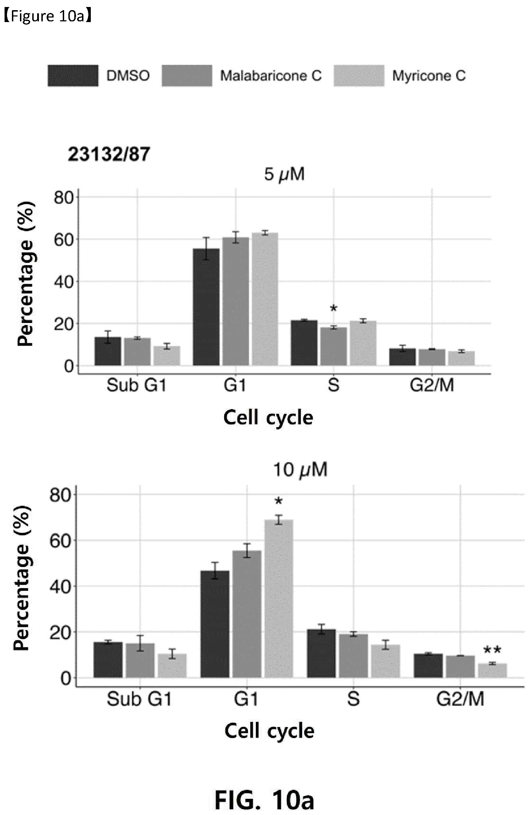

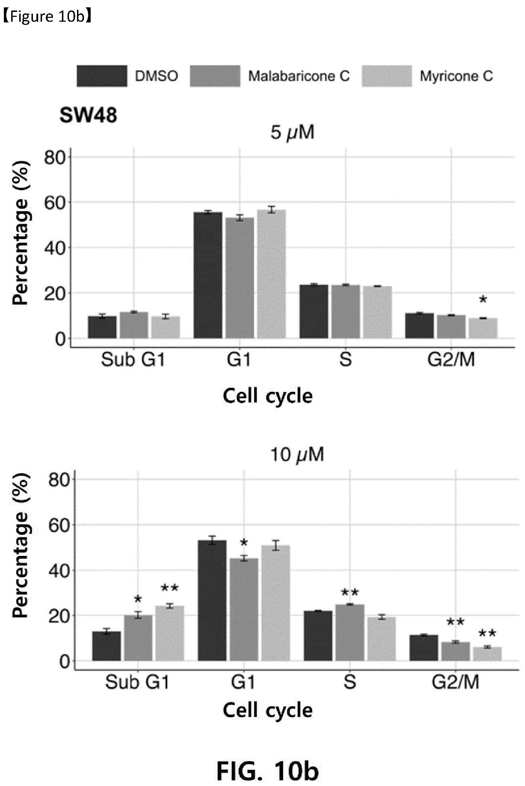

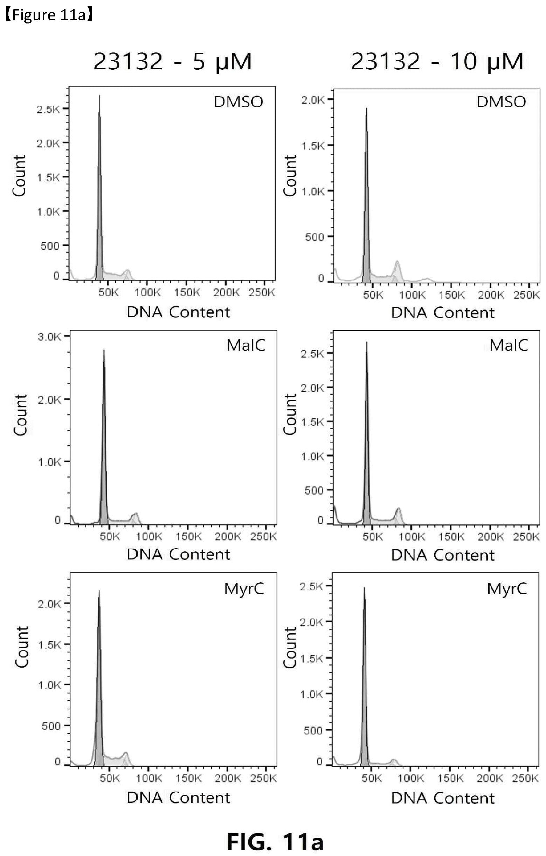

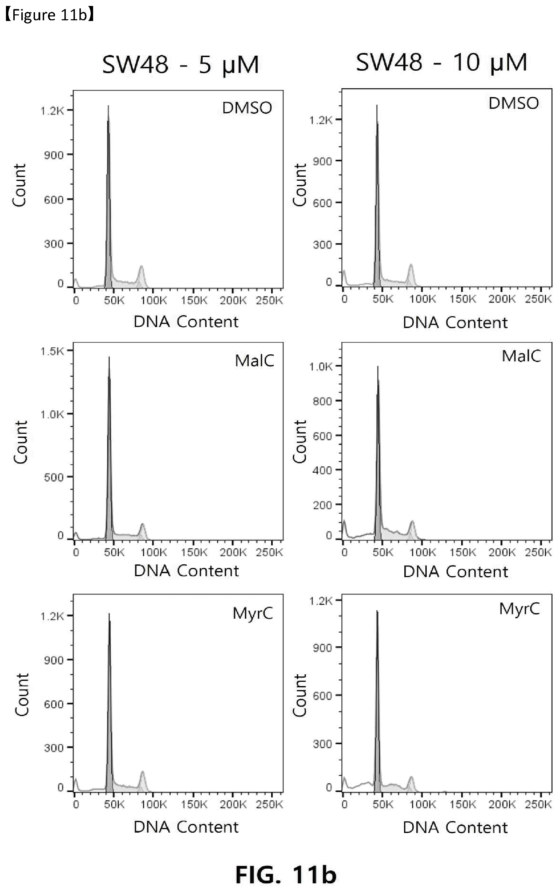

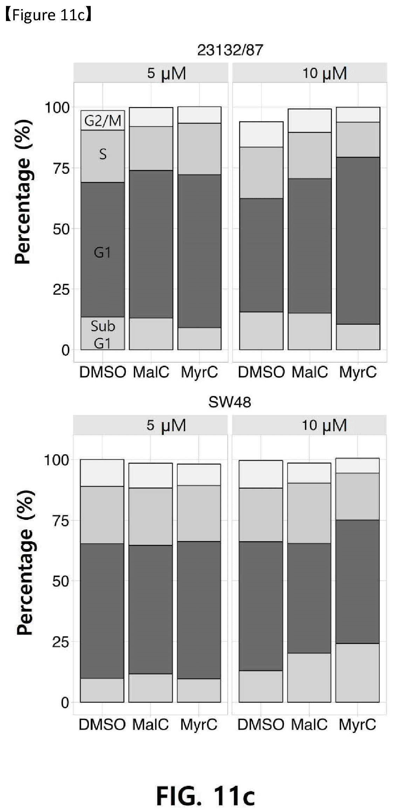

The results, as shown in Figures 10 and 11, indicated that overall, the 10 µM compound significantly disrupted the cell cycle in both tested cell lines, unlike the 5 µM compound.

Further, comparing the differences between samples treated with each compound and the DMSO-only control group, for the 23132/87 cells, Myricone C (10 µM) arrested the cell cycle at the G1 phase, increasing it by about 10% compared to DMSO. Inevitably, the proportions of the remaining phases decreased, with the G2/M phase decreasing by about 4%.

In contrast, Malabaricone C did not have a noticeable effect on these gastric cancer cells at either concentration. For SW48 cells, treatment with 10 µM Myricone C slightly reduced the proportions of the G1, S, and G2/M phases, but only the G2/M phase showed a statistically significant decrease. Meanwhile, Malabaricone C increased the proportion of the S phase by about 2% and decreased the G1 and G2/M phases by about 8% and 3%, respectively. Notably, Myricone C and Malabaricone C at 10 µM increased the sub-G1 proportion by 7% and 11%, respectively, compared to the DMSO control.

### 3-2: Cell Cycle Distribution Analysis by Myricone C Concentration

To examine the cell cycle distribution based on Myricone C concentration, cells were treated with Myricone C and Malabaricone C at 5 µM and 10 µM, followed by analysis of the cell cycle distribution in the 23132/87 and SW48 cell lines. Student's t-test was performed for statistical analysis between the 2 concentrations of the same compound.

**[Table 20]**

| Cell cycle distribution upon treatment with Myricone C and Malabaricone C compounds | | | | | | |
|---|---|---|---|---|---|---|
| Cell line | Compound | Cycle | 5 µM | | 10 µM | |
| | | | % | SD | % | SD |
| 23132/87 | Malabaricone C | Sub G1 | 13.07 | 0.55 | 15.08 | 3.36 |
| | | G1 | 60.87 | 2.67 | 55.47 | 3.03 |
| | | S | 18.10 | 0.72 | 19.07 | 0.99 |
| | | G2/M | 7.78 | 0.17 | 9.63 | 0.08 |
| | Myricone C | Sub G1 | 9.15 | 1.31 | 10.45 | 2.08 |
| | | G1 | 63.00 | 1.05 | 68.93 | 1.96 |
| | | S | 21.23 | 0.94 | 14.40 | 1.99 |
| | | G2/M | 6.79 | 0.69 | 6.19 | 0.47 |
| SW48 | Malabaricone C | Sub G1 | 11.63 | 0.38 | 20.17 | 1.47 |
| | | G1 | 53.10 | 1.27 | 45.27 | 1.19 |
| | | S | 23.53 | 0.32 | 24.83 | 0.33 |
| | | G2/M | 10.21 | 0.24 | 8.30 | 0.49 |
| | Myricone C | Sub G1 | 9.65 | 1.02 | 24.17 | 0.98 |
| | | G1 | 56.63 | 1.44 | 50.93 | 2.17 |
| | | S | 23.00 | 0.15 | 19.30 | 0.98 |
| | | G2/M | 8.91 | 0.24 | 6.13 | 0.47 |

As a result, as shown in Table 20 above, treatment with the compound at a concentration of 10 µM has been found to be more suitable for SW48 cell line therapy. Specifically, increasing the concentration of Myricone C from 5 µM to 10 µM significantly increased the sub-G1 phase ratio from 9.65% ± 1.02% to 24.17% ± 0.98%, while the G2 phase decreased from 8.91% ± 0.24% to 6.13% ± 0.47%. Similarly, Malabaricone C at 10 µM affected all phases of the cell cycle more than at 5 µM. That is, Myricone C at 10 µM has been confirmed to significantly affect the cell cycle of colorectal cancer SW48 cells.

Malabaricone C increased the proportion of cells in the S and sub-G1 phases while suppressing the remaining phases, whereas Myricone C reduced the G2/M phase and shifted cells into the sub-G1 phase. For gastric cancer cell line 23132/87, both selected compounds showed an upward trend in the G1 phase, but an opposite trend was observed in the S and G2/M phases.

### Example 4: Induction of Apoptosis in Cancer Cells by Myricone C Compound

### 4-1: Measurement of Apoptosis

In the present invention, to confirm whether apoptosis is induced in cancer cells by the Myricone C compound, apoptosis was measured in cells treated with the compound using the Annexin V-FITC/PI staining method.

23132/87 and SW48 cells were seeded at densities of 5 × 10⁵ and 7 × 10⁵ cells/well in a 6-well plate and cultured for 24 hours. Next, the cells were treated with 2 concentrations of Myricone C or Malabaricone C (5 and 10 µM) or the control DMSO for 20 hours. After treatment, cells were harvested and stained using the Annexin V-FITC Early Apoptosis Detection Kit (6592, Cell Signaling Technology, USA). Flow cytometry (BD FACSVerse Cell Analyzer and FlowJo software, BD Biosciences, USA) was applied to analyze the samples based on 10,000 events.

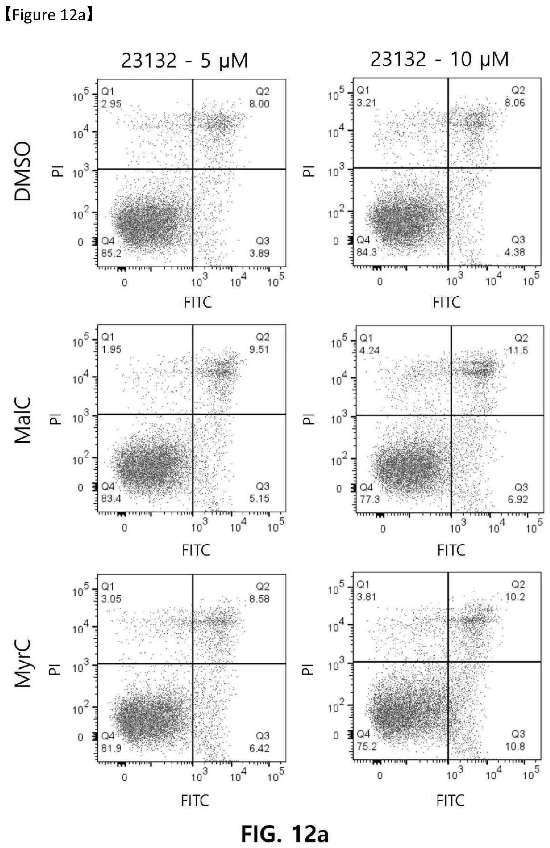

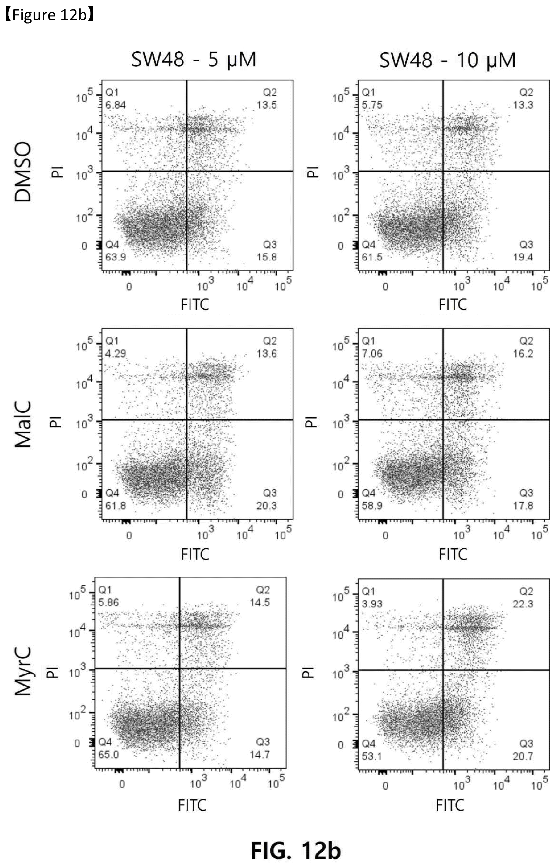

The results, as shown in Figure 12, indicated that compound treatment induced signs of apoptosis in gastric and colorectal cancer cell lines, as evidenced by increased Annexin V-FITC+/PI+ (Q2, late apoptosis and cell death) and Annexin V-FITC+/PI- (Q3, early apoptosis) populations, while decreasing the healthy population Annexin V-FITC-/PI- (Q4).

### 4-2: Comparison of apoptosis-related cell populations (Q2 and Q3) with the control group

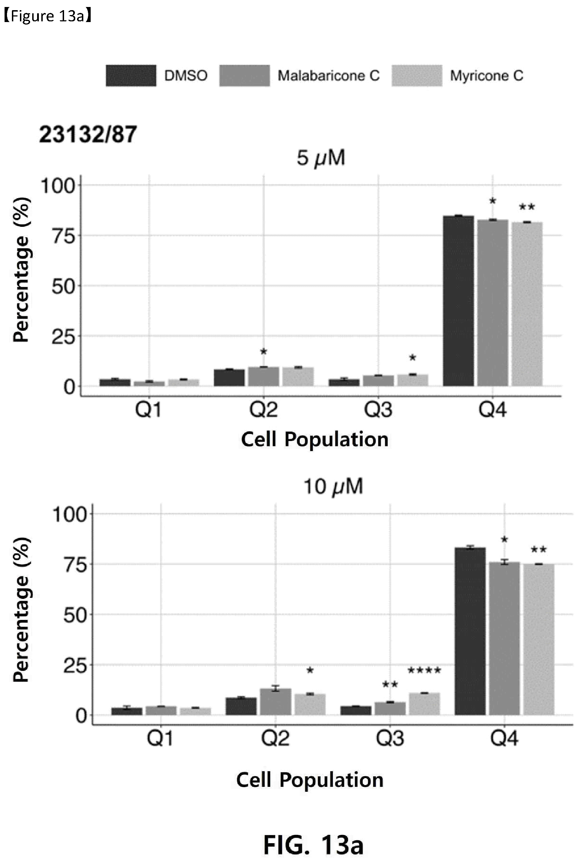

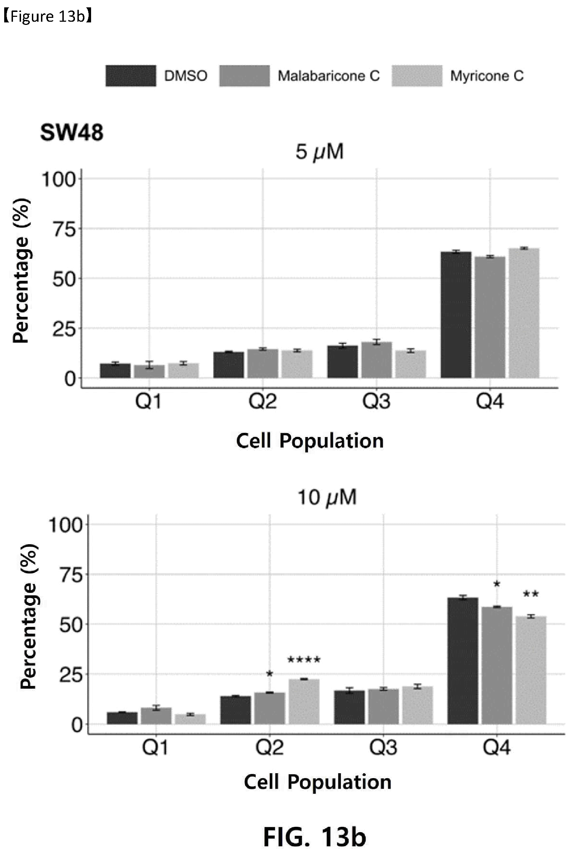

As shown in Figure 13, comparing the Annexin V-FITC-positive cell populations Q2 and Q3 in treated samples with the DMSO control, Malabaricone C statistically significantly increased the Q2 population at 5 µM and the Q3 population at 10 µM in the 23132/87 cell line. Similarly, Myricone C significantly increased the Q3 ratio at 5 µM and increased the Q2 and Q3 ratios by about 2% and 6%, respectively, at 10 µM. No significant differences were observed in SW48 cells at 5 µM treatment. However, at the higher 10 µM concentration, Annexin V-FITC+/PI+ signals clearly increased, particularly for Myricone C, where Q2 reached 22.43% ± 0.30%, representing an increase of over 8% compared to the control.

### 4-3: Analysis of Intercellular Differences

To confirm the cell cycle distribution according to the Myricone C treatment concentration, Myricone C and Malabaricone C compounds was treated at 5 µM and 10 µM, followed by analysis of the cell cycle distribution in the 23132/87 and SW48 cell lines. Student's t-test was performed for statistical analysis between the 2 concentrations of the same compound.

**[Table 21]**

| Apoptosis distribution (%) upon treatment with Myricone C and Malabaricone C compounds | | | | | | |
|---|---|---|---|---|---|---|
| Cell line | Compound | Area | 5 µM | | 10 µM | |
| | | | % | SD | % | SD |
| 23132/87 | Malabaricone C | Q1 | 2.35 | 0.37 | 4.33 | 0.10 |
| | | Q2 | 9.54 | 0.12 | 13.23 | 1.40 |
| | | Q3 | 5.36 | 0.12 | 6.45 | 0.30 |
| | | Q4 | 82.77 | 0.35 | 76.00 | 1.20 |
| | Myricone C | Q1 | 3.30 | 0.24 | 3.59 | 0.17 |
| | | Q2 | 9.35 | 0.40 | 10.47 | 0.42 |
| | | Q3 | 5.76 | 0.35 | 10.97 | 0.12 |
| | | Q4 | 81.57 | 0.28 | 75.00 | 0.20 |
| SW48 | Malabaricone C | Q1 | 6.51 | 1.81 | 8.12 | 1.14 |
| | | Q2 | 14.50 | 0.59 | 15.70 | 0.25 |
| | | Q3 | 18.10 | 1.30 | 17.53 | 0.65 |
| | | Q4 | 60.90 | 0.55 | 58.63 | 0.37 |
| | Myricone C | Q1 | 7.41 | 0.83 | 4.97 | 0.52 |
| | | Q2 | 13.80 | 0.65 | 22.43 | 0.30 |
| | | Q3 | 13.73 | 0.92 | 18.77 | 1.07 |
| | | Q4 | 65.10 | 0.49 | 53.87 | 0.77 |

As shown in Table 21 above, when the compound treatment concentration was doubled, Myricone C increased the Q3 population of 23132/87 cells from 5.76% ± 0.35% to 10.97% ± 0.12%, and increased the Q2 and Q3 ratios in SW48 cells from 13.80% ± 0.65% to 22.43% ± 0.30% and from 13.73% ± 0.92% to 18.77% ± 1.07%, respectively. The increase in the Q2 population of SW48 cells by over 8% was the highest among all dose-based comparisons in the experiment, indicating the potent effect of Myricone C. Notably, changes in cell distribution induced by DMSO were not statistically significant even when the concentration was doubled in both cell lines.

### 4-4: Comparison of Apoptosis-Related Cell Populations (Q2 and Q3) and Non-Apoptotic Cell Population (Q4)

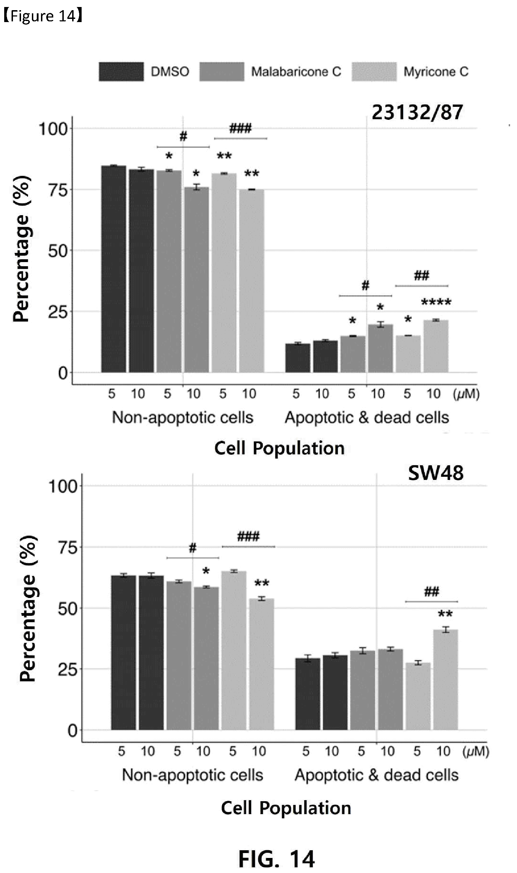

To more clearly visualize the comparison between apoptosis-related cell populations (Q2 and Q3) and the non-apoptotic cell population (Q4), a side-by-side analysis was performed. As shown in Figure 14, the healthy population was significantly reduced in all treatments compared to the control group in both cell lines. Specifically, 10 µM Myricone C most significantly reduced the Q4 distribution by 8.27% and 9.43% in 23132/87 and SW48 cells, respectively.

Further, the Q4 ratio decreased consistently with increasing concentration. Notably, in SW48 cells treated with Myricone C, changing the concentration from 5 µM to 10 µM resulted in a reduction of approximately 11.23%.

The apoptosis-related group includes early apoptotic cells (Q3), late apoptotic cells, and dead cells (Q2). Q2 and Q3 significantly increased with increasing concentrations in 23132/87 cells treated with the Myricone C compound. In SW48 cells, Malabaricone C showed a trend toward increasing the apoptotic and dead cell populations, but this was not statistically significant. However, 10 µM Myricone C increased this population nearly 10% more effectively than DMSO.

Thus, apoptosis analysis using Annexin V/FITC-PI double staining confirmed that the Myricone C compound can induce apoptosis in both gastric and colorectal cancer cell lines, with 10 µM being the preferred concentration.

### Example 5: Effect of Myricone C Compound on Caspase-3/7 Activity in Cancer Cells

Apoptosis can be activated and progress through either the intrinsic or extrinsic pathway, but caspase-3, the executioner caspase, is involved in most cases. Therefore, caspase activity analysis was performed to evaluate the real-time activity of caspase-3 and -7 in cells treated with Myricone C and Malabaricone C compounds. Since this enzyme activity is transient, analysis was performed at two time points: 6 hours and 12 hours post-treatment.

Specifically, the Caspase-3/7 activity assay was performed with slight modifications to the manufacturer's protocol (Promega, USA). Briefly, cells were seeded at a density of 5 × 10³ cells/well in a 96-well plate, cultured for 24 hours, and then treated with the compound at an appropriate concentration for 6 or 12 hours. After treatment, Caspase-Glo 3/7 reagent was added in equal volumes to the treated samples. The plate was then shaken and incubated at room temperature for 2 hours. Enzyme activity was determined by measuring relative light units (RLU) using a Varioskan LUX Multimode Microplate Reader (Thermo Fisher Scientific, USA).

**[Table 22]**

| Caspase-3/7 activity upon treatment with Myricone C and Malabaricone C compounds | | | | | | | | |
|---|---|---|---|---|---|---|---|---|
| | 23132/87 | | | | SW48 | | | |
| | MalC | | MyrC | | MalC | | MyrC | |
| | 5 µM | 10 µM | 5 µM | 10 µM | 5 µM | 10 µM | 5 µM | 10 µM |
| 6 h | 0.43 | 0.33 | 0.58 | 1.81 | 0.80 | 0.68 | 1.10 | 1.89 |
| 12 h | 0.33 | 0.34 | 1.74 | 1.20 | 1.20 | 0.57 | 2.37 | 2.02 |

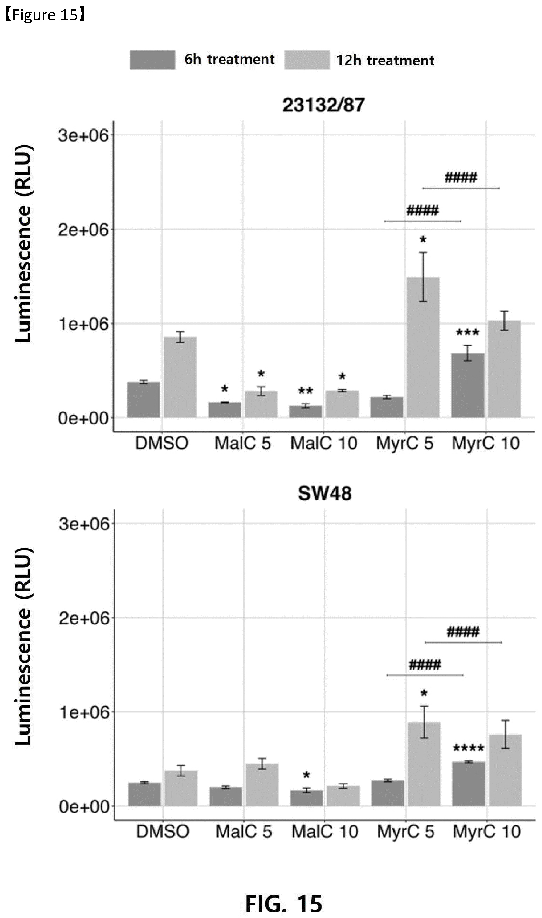

As shown in Figure 15 and Table 22, for gastric cancer cells, Malabaricone C showed significantly lower relative light units (RLU) than the DMSO control at all concentrations. 5 µM Myricone C exhibited its highest peak after 12 hours, while 10 µM showed its peak only after 6 hours, representing 1.74-fold and 1.81-fold increases over DMSO, respectively.

For colorectal cancer cells, low concentrations of Malabaricone C showed signals similar to DMSO at both time points. At 10 µM, a significantly lower signal was observed after 6 hours, while after 12 hours, the signal was similar to the control. Myricone C showed a similar trend to gastric cancer cells, with signals increasing markedly over time from 6 to 12 hours at the same concentrations. However, low concentrations peaked only after 12 hours, while high concentrations peaked after 6 hours. These values were 1.89-fold and 2.37-fold higher than DMSO, respectively.

Based on the above evidence and the fact that RLU values correlate with caspase-3/7 activity, it can be concluded that Myricone C (5 µM - 10 µM) significantly increased caspase activity and induced apoptosis in 23132/87 and SW48 cells between 6 and 12 hours. In contrast, Malabaricone C did not affect this enzymatic reaction under the test conditions.

### Example 6: Confirmation of Anticancer Effects Following Administration of Myricone C Compound in a Xenograft Mouse Model

Female BALB/c/nu/nu mice (5 weeks old) were used in the in vivo model.

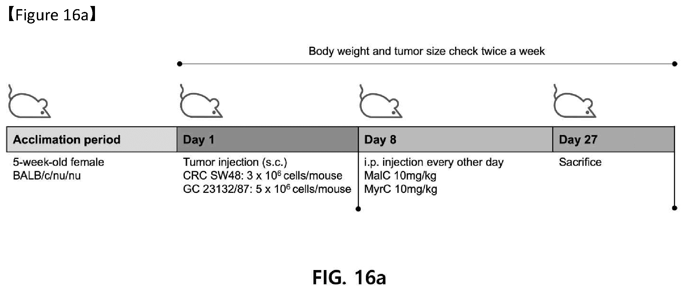

As shown in the schematic diagram of Figure 16a, 23132/87 and SW48 cells were injected subcutaneously into mice at densities of 5 × 10⁶ cells/mouse and 3 × 10⁶ cells/mouse, respectively. Once tumors reached a minimum width of 3 mm, mice were divided into 3 groups and received intraperitoneal injections of Myricone C (10 mg/kg), Malabaricone C (10 mg/kg), or the control DMSO (2% in saline solution) on alternate days. Tumor volume and body weight were measured twice weekly. On day 27, mice were sacrificed, and major organs (liver, spleen, kidney) and tumor tissue were collected and stored for further analysis. Tumor volume was calculated using the following formula: Volume (mm³) = (Length) × (Width)² × 1/2.

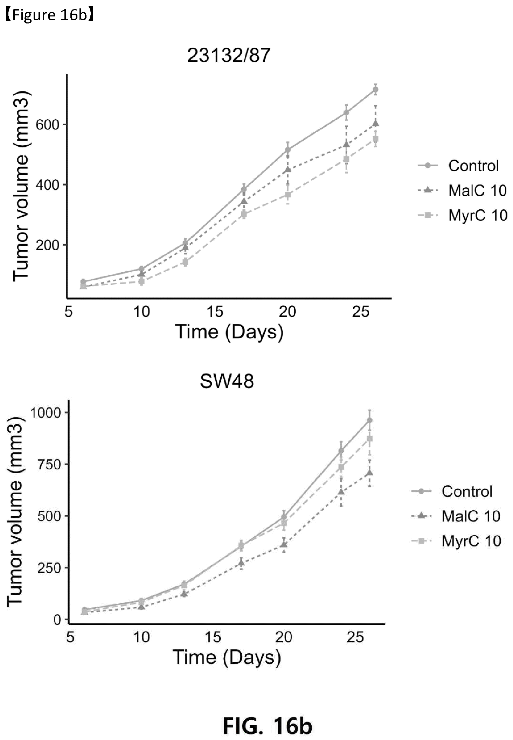

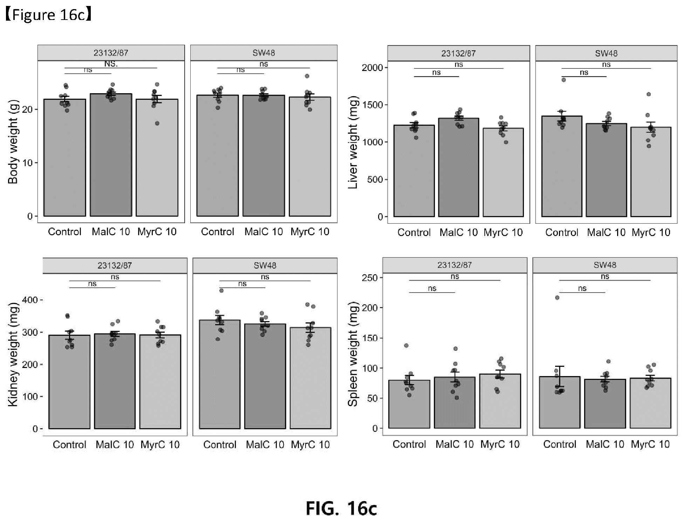

Monitoring tumor volume and body weight throughout the experiment revealed that tumor volume gradually increased in all groups. However, for gastric cancer, the smallest tumor volume was observed starting on day 13 of Myricone C administration. For colorectal cancer, tumor growth was also suppressed compared to the control group (Figure 16b). Furthermore, body weight and the weights of major organs, including the liver, spleen, and kidneys, remained stable throughout the experimental period (Figure 16c).

### [INDUSTRIAL APPLICABILITY]

The novel compounds isolated in this invention have been confirmed to exhibit cytotoxic effects against various cancer cell lines. Five compounds (3, 4, 6, 9, and 11) have been found to show high cytotoxic effects in gastric and colorectal cancers. In particular, the compound represented by Formula 3 was found not only to effectively induce apoptosis through regulation of the cancer cell cycle but also to effectively suppress tumor growth in xenograft mouse models, making it useful as an anticancer composition.

## Claims

1. A compound represented by the following Formula A, a pharmaceutically acceptable salt thereof, or an optical isomer thereof: 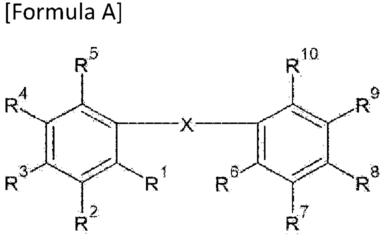
wherein, in Formula A,
X is 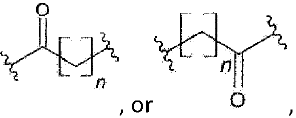 wherein n is an integer of 1 to 20;
at least one of R¹ to R⁵ is 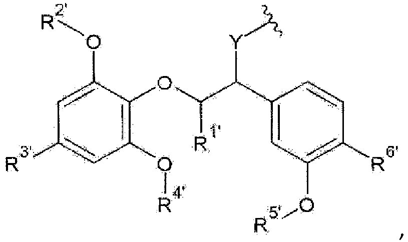 wherein Y is O or - (CH)m- (where m is 0 to 5), and R^{1'} to R^{6'} are each independently hydrogen, hydroxy, optionally substituted C₁₋₆ alkyl, optionally substituted C₁₋₆ alkoxy, optionally substituted C₃₋₈ cycloalkyl, optionally substituted C₂₋₁₀ allyl, or optionally substituted C₃₋₁₀ heterocyclic group;
the remaining R¹ to R⁵ are each independently hydrogen, hydroxy, optionally substituted C₁₋₆ alkyl, optionally substituted C₁₋₆ alkoxy, optionally substituted C₃₋₈ cycloalkyl, optionally substituted C₂₋₁₀ allyl, or optionally substituted C₃₋₁₀ heterocyclic group;
R⁶, R⁸ and R¹⁰ are each independently hydrogen or hydroxy;
R⁷ and R⁹ are each independently hydrogen; and
R^{1'} to R^{6'} are each independently hydrogen, hydroxy, optionally substituted C₁₋₆ alkyl, optionally substituted C1-6 alkoxy, optionally substituted C₃₋₈ cycloalkyl, optionally substituted _{C2-10} allyl, or optionally substituted C₃₋₁₀ heterocyclic group.

2. The compound of claim 1, wherein the compound represented by the above Formula A is a compound represented by the following Formula 3; a compound represented by the following Formula 4; a compound represented by the following Formula 6; a compound represented by the following Formula 9; a compound represented by the following Formula 10; a compound represented by the following Formula 11; a compound represented by the following Formula 12; or a compound represented by the following Formula 13:

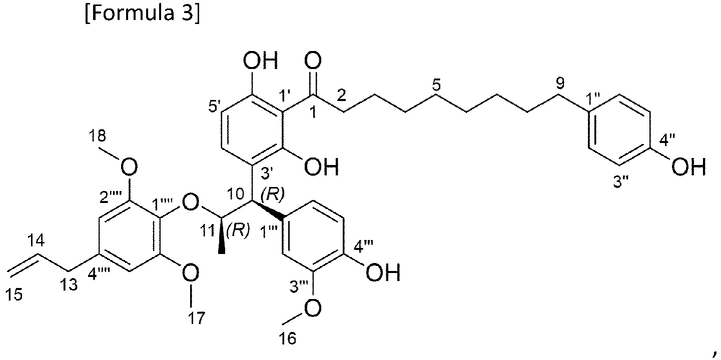

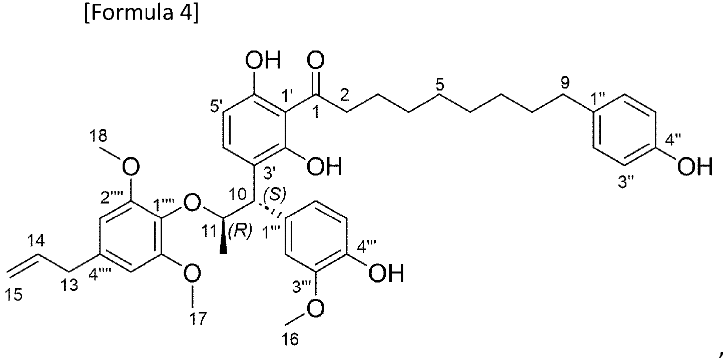

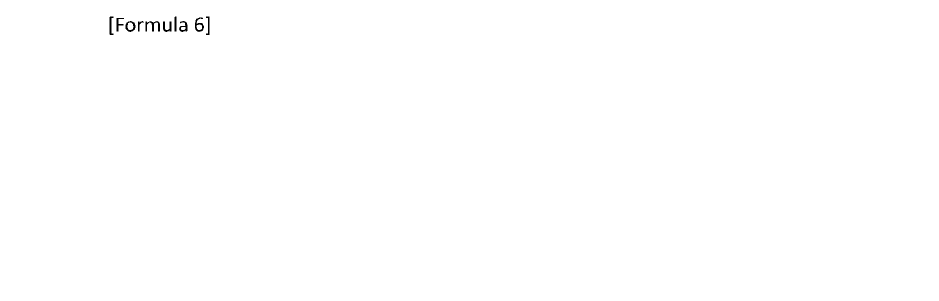

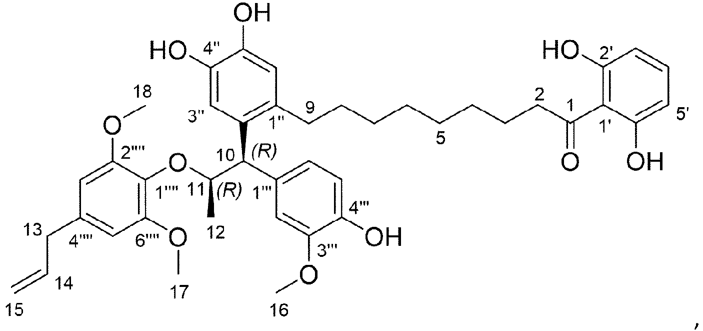

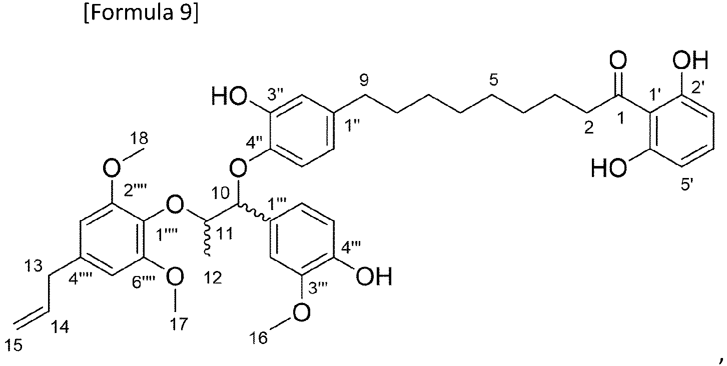

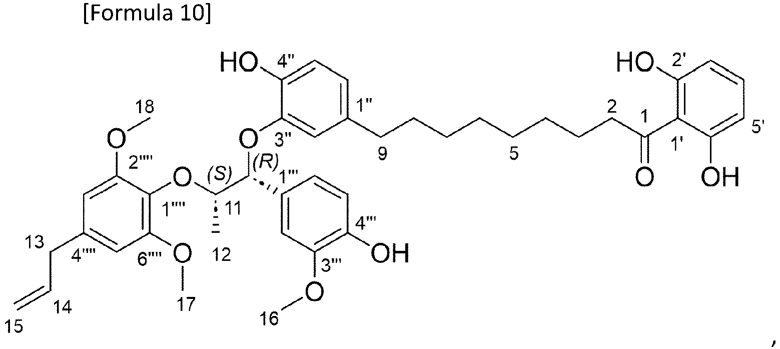

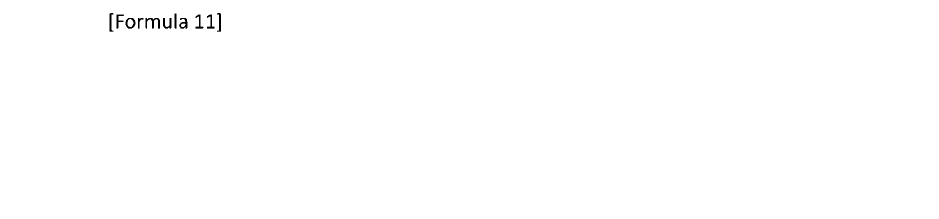

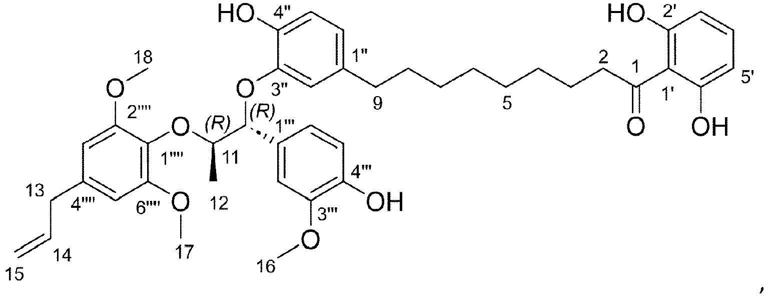

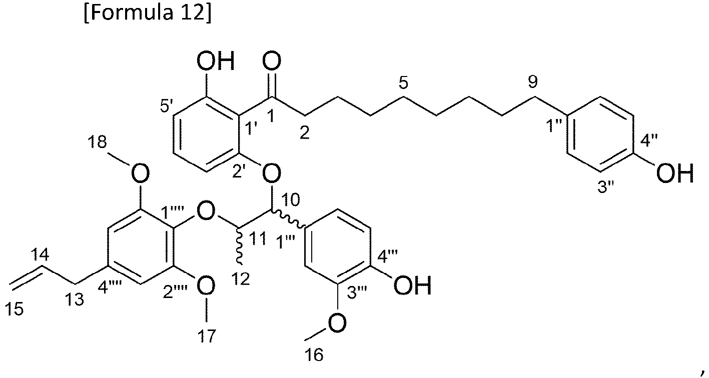

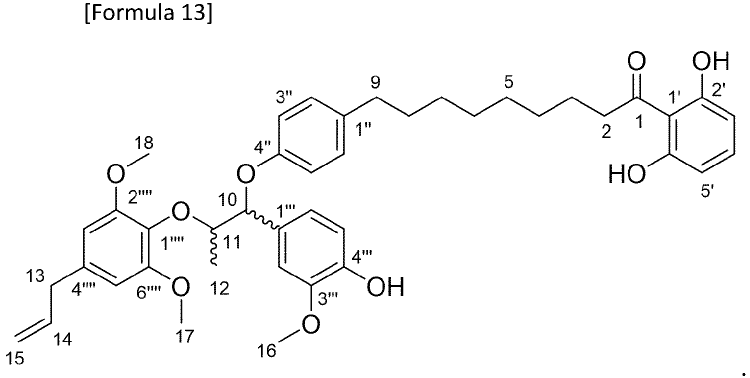

3. The compound of claim 1, wherein the compound represented by the above Formula A is a compound represented by the following Formula 3:

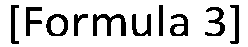

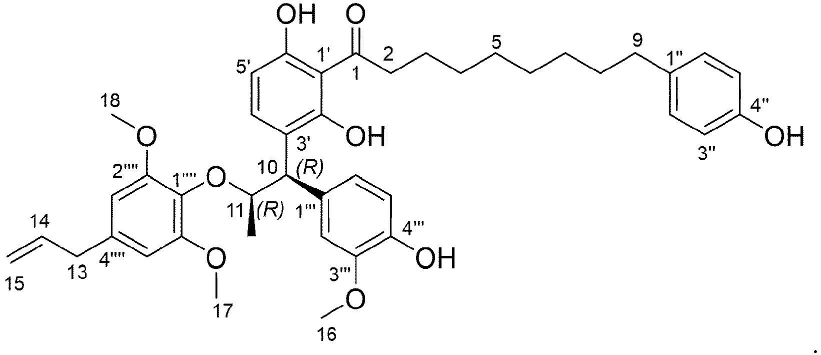

4. A compound represented by the following Formula 14, a pharmaceutically acceptable salt thereof, or an optical isomer thereof; a compound represented by the following Formula 15, a pharmaceutically acceptable salt thereof, or an optical isomer thereof; or a compound represented by the following Formula 16, a pharmaceutically acceptable salt thereof, or an optical isomer thereof:

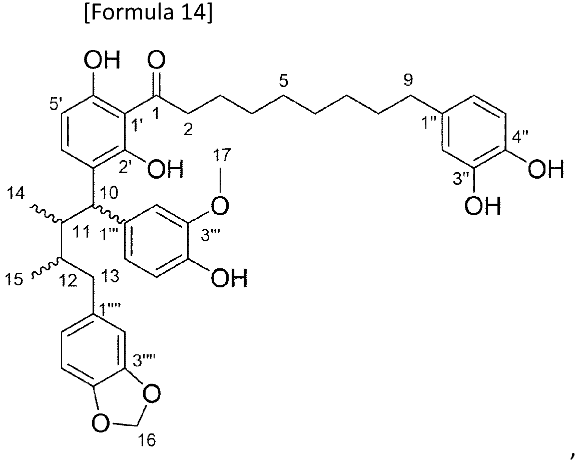

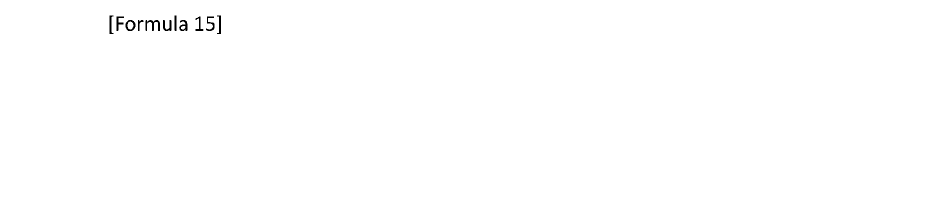

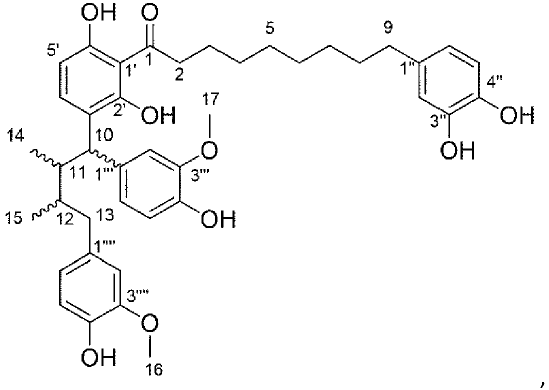

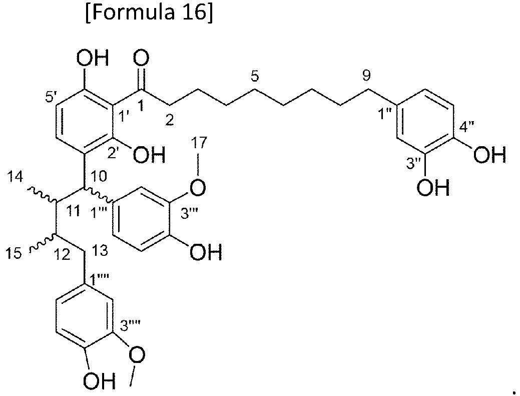

5. A compound represented by the following Formula 17, a pharmaceutically acceptable salt thereof, or an optical isomer thereof; a compound represented by the following Formula 18, a pharmaceutically acceptable salt thereof, or an optical isomer thereof; a compound represented by the following Formula 19, a pharmaceutically acceptable salt thereof, or an optical isomer thereof; or a compound represented by the following Formula 20, a pharmaceutically acceptable salt thereof, or an optical isomer thereof:

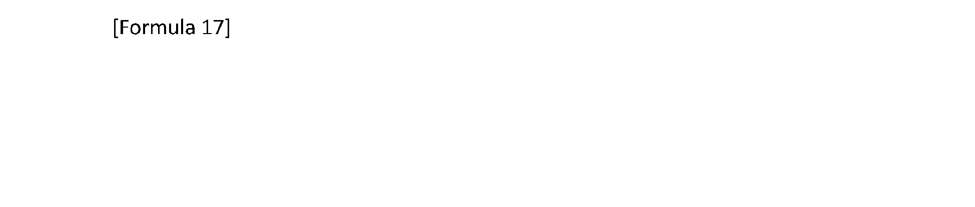

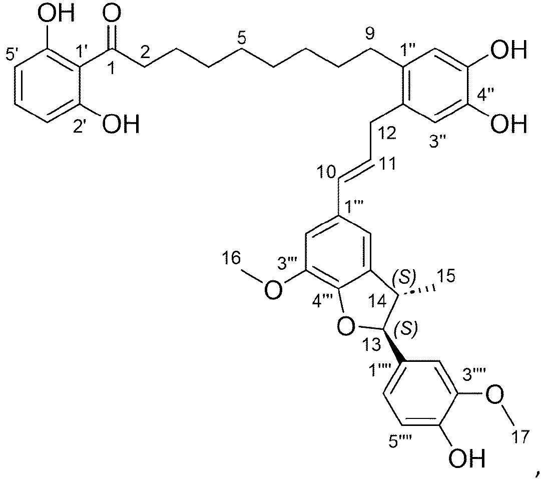

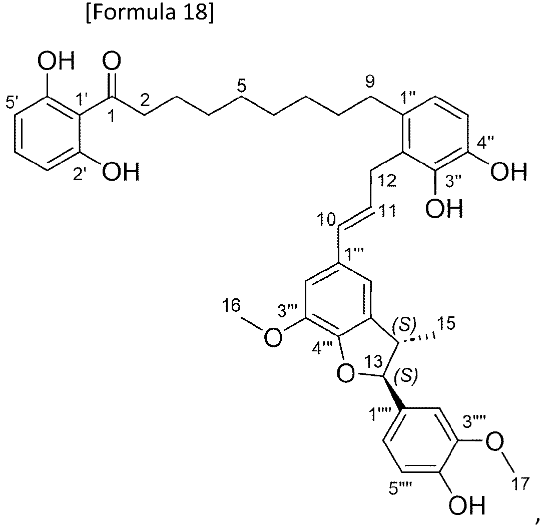

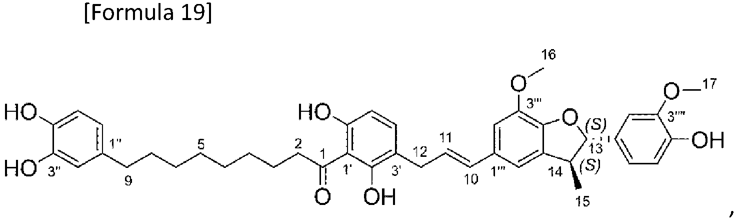

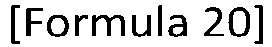

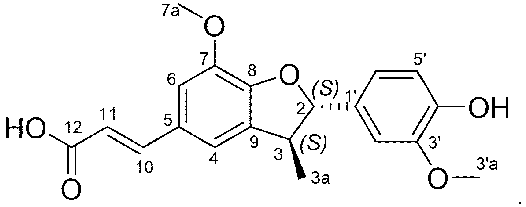

6. A compound represented by the following Formula 21, a pharmaceutically acceptable salt thereof, or an optical isomer thereof; or a compound represented by the following Formula 22, a pharmaceutically acceptable salt thereof, or an optical isomer thereof:

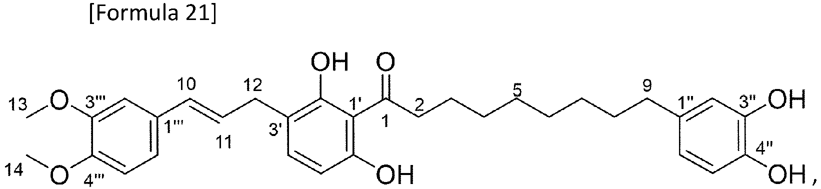

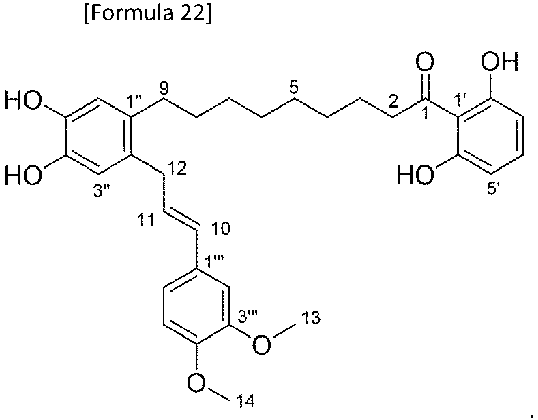

7. A pharmaceutical composition for preventing or treating cancer, comprising the compound according to any one of Claims 1 to 6 as an active ingredient.

8. The pharmaceutical composition of Claim 7, wherein the cancer is pancreatic cancer, liver cancer, lung cancer, stomach cancer, colorectal cancer, prostate cancer, kidney cancer, breast cancer, or ovarian cancer.

9. A health functional food composition for cancer prevention or improvement, comprising the compound according to any one of Claims 1 to 6 as an active ingredient.

10. The health functional food composition of Claim 9, wherein the cancer is pancreatic cancer, liver cancer, lung cancer, stomach cancer, colorectal cancer, prostate cancer, kidney cancer, breast cancer, or ovarian cancer.
